# EUROPEAN PATENT SPECIFICATION

(11) **EP 2 794 651 B1**
(45) Date of publication and mention of the grant of the patent: **21.09.2022**
(21) Application number: 12810245.6
(22) Date of filing: 19.12.2012
(51) Int. Cl.: C07K 16/00, C12N 15/67

(54) **EXPRESSION VECTOR ELEMENT COMBINATIONS, NOVEL PRODUCTION CELL GENERATION METHODS AND THEIR USE FOR THE RECOMBINANT PRODUCTION OF POLYPEPTIDES**
EXPRESSIONSVEKTORELEMENTKOMBINATIONEN, NEUE VERFAHREN ZUR PRODUKTIONSZELLENERZEUGUNG UND DEREN VERWENDUNG BEI DER REKOMBINANTEN HERSTELLUNG VON POLYPEPTIDEN
COMBINAISONS D'ÉLÉMENTS DE VECTEUR D'EXPRESSION, NOUVEAUX PROCÉDÉS DE GÉNÉRATION DE CELLULES DE PRODUCTION ET LEUR UTILISATION POUR LA PRODUCTION RECOMBINANTE DE POLYPEPTIDES

(30) Priority: 22.12.2011 EP 11195361
(43) Date of publication of application: 29.10.2014
(60) Divisional application: 18157484.9
(73) Proprietor: F. Hoffmann-La Roche AG, 4070 Basel (CH)
(72) Inventor: HUELSMANN, Peter Michael, 82392 Habach (DE); KNOETGEN, Hendrik, 79650 Schopfheim (DE)
(74) Representative: Burger, Alexander
(86) International application number: PCT/EP2012/076203
(87) International publication number: WO 2013/092743

(56) References cited:
- WO-A1-2010/097437
- SANNA PIETRO P: "EXPRESSION OF ANTIBODY FAB FRAGMENTS AND WHOLE IMMUNOGLOBULIN IN MAMMALIAN CELLS", METHODS IN MOLECULAR BIOLOGY, HUMANA PRESS INC, NJ, US, vol. 178, 1 January 2002 (2002-01-01), pages 389-395, XP009083985, ISSN: 1064-3745 cited in the application
- YEW N S ET AL: "OPTIMIZATION OF PLASMID VECTORS FOR HIGH-LEVEL EXPRESSION IN LUNG EPITHELIAL CELLS", HUMAN GENE THERAPY, MARY ANN LIEBERT, NEW YORK ,NY, US, vol. 8, no. 5, 20 March 1997 (1997-03-20), pages 575-584, XP000940966, ISSN: 1043-0342
- D. KIM ET AL: "Improved Mammalian Expression Systems by Manipulating Transcriptional Termination Regions", BIOTECHNOLOGY PROGRESS, vol. 19, no. 5, 3 October 2003 (2003-10-03), pages 1620-1622, XP055030207, ISSN: 8756-7938, DOI: 10.1021/bp0341186
- WINNARD PAUL JR ET AL: "Robust expression of transgenes in MCF-7 breast cancer cells is expression vector-dependent", BIOTECHNIQUES, vol. 37, no. 3, September 2004 (2004-09), XP002699747, ISSN: 0736-6205
- STEVEN C L HO ET AL: "IRES-mediated Tricistronic vectors for enhancing generation of high monoclonal antibody expressing CHO cell lines", JOURNAL OF BIOTECHNOLOGY, ELSEVIER SCIENCE PUBLISHERS, AMSTERDAM, NL, vol. 157, no. 1, 19 September 2011 (2011-09-19), pages 130-139, XP028395739, ISSN: 0168-1656, DOI: 10.1016/J.JBIOTEC.2011.09.023 [retrieved on 2011-10-17]
- LI ET AL: "A comparative study of different vector designs for the mammalian expression of recombinant IgG antibodies", JOURNAL OF IMMUNOLOGICAL METHODS, ELSEVIER SCIENCE PUBLISHERS B.V.,AMSTERDAM, NL, vol. 318, no. 1-2, 3 January 2007 (2007-01-03), pages 113-124, XP005820142, ISSN: 0022-1759, DOI: 10.1016/J.JIM.2006.10.010
- JIN-CHING LEE ET AL: "High-efficiency protein expression mediated by enterovirus 71 internal ribosome entry", BIOTECHNOLOGY AND BIOENGINEERING, WILEY & SONS, HOBOKEN, NJ, US, vol. 90, no. 5, 5 June 2005 (2005-06-05), pages 656-662, XP002677796, ISSN: 0006-3592, DOI: 10.1002/BIT.20440 [retrieved on 2005-04-07]
- HOTTA A ET AL: "Production of anti-CD2 chimeric antibody by recombinant animal cells", JOURNAL OF BIOSCIENCE AND BIOENGINEERING, ELSEVIER, AMSTERDAM, NL, vol. 98, no. 4, 1 January 2004 (2004-01-01), pages 298-303, XP004649520, ISSN: 1389-1723

## Description

Herein are reported novel combinations of expression vector elements, such as promoter, polyA signal sequence, and transcription terminator, expression vector organizations, combinations thereof, as well as novel methods for the generation of production cell lines, such as novel transfection or selection methods, as well as the use of these expression vectors and production cell lines for the recombinant production of polypeptides of interest.

### Background of the Invention

The transcription level of a gene can have a strong influence on its expression level and therefore determines the productivity of a cell. It is mainly influenced by three vector elements: by the promoter, the polyA signal sequence and (if present) by a transcription terminator.

The nucleic acid encoding an antibody heavy chain generally comprises a leader sequence (a signal sequence) (approximately 57 bp/19 aa), which is removed upon maturation of the protein, a variable region, VH (approximately 350 bp/115 aa), and the constant region, CH (approximately 990 bp/330 aa). The nucleic acid encoding an antibody light chain is generally composed of a leader sequence (approximately 66 bp/22 aa) which is removed upon maturation of the protein, a variable region, VK or VL (approximately 350 bp/115 aa), and a constant region, CK or CL (approximately 321 bp/107 aa).

The recombinant production of antibodies in eukaryotic cells involves the creation of expression systems (see, McCafferty, J., et al., (eds.), Antibody Engineering, A Practical Approach., IRL Press (1997)). For development of antibody expression systems an expression cassette comprising a light chain encoding nucleic acid flanked by a promoter and a poly-adenylation (polyA) region is created. Also, a heavy chain expression cassette comprising a heavy chain encoding nucleic acid flanked by a promoter and a polyA region is created. The expression cassette of the heavy chain may be combined into the light chain expression cassette in a single vector containing both heavy and light chain expression cassettes or may be integrated in two separate vectors.

Immunoglobulin DNA cassette molecules, monobody constructs, methods of production, and methods of use therefor are reported in US 7,053,202. In US 5,168,062 transfer vectors and microorganisms containing human cytomegalovirus immediate-early promoter-regulatory DNA sequence are reported. A DNA fragment containing a promoter region for human polypeptide chain elongation factor-lα, its base sequence and expression plasmids containing the DNA fragment having high applicability to a wide range of host cells with high expression capacity is reported in US 5,225,348. In US 5,266,491 expression plasmids containing SV40 replication origin and a DNA fragment having a promoter region for a human polypeptide chain elongation factor-lα gene are reported. Recombinant DNA compounds and the expression of polypeptides such as tPA are reported in US 5,122,458. In US 7,422,874 an expression vector for animal cell is reported.

Sanna Pietro, P., reports the expression of antibody Fab fragments and whole immunoglobulin in mammalian cells (Meth. Mol. Biol. 178 (2002) 389-395). A cell display library for gene cloning of variable regions of human antibodies to hepatitis B surface antigen is reported by Higuchi, K., et al. (J. Immunol. Meth. 202 (1997) 193-204). Kim, D., et al., report improved mammalian expression systems by manipulating transcriptional termination regions (Biotechnol. Progress 19 (2003) 1620-1622). Guidelines to cell engineering for monoclonal antibody production are reported by Costa, R.A., et al. (Eur. J. Pharmaceut. Biopharmaceut. 74 (2010) 127-138). Kim, D.W., et al., report the use of the human elongation factor 1 alpha promoter as a versatile and efficient expression system (Gene 91 (1990) 217-223). Comparison of intron-dependent and intron independent gene expression is reported by Buchman, A.R., et al., (Mol. Cell. Biol. 8 (1988) 4395-4405). Wang, F., et al., report antibody expression in mammalian cells (in Therapeutic monoclonal antibodies - From bench to clinic, Wiley (2009) pages 557-572). A comparative study of different vector designs for the mammalian expression of recombinant lgG antibodies is reported by Li et al. (J. Immunol. Meth. 318 (2007) 113-124). Ho, S.C.L., et al. report "IRES-mediated tricistronic vectors for enhancing generation of high monoclonal antibody expressing CHO cell lines (J. Biotechnol. 157 (2011) 130-139). Production of anti-CD2 chimeric antibody by recombinant animal cells are reported by Hotta, A., et al. (J. Biosci. Bioeng. 98 (2004) 298-303). Lee, J-C., et al. report "High-efficiency protein expression mediated by enterovirus 71 internal ribosome entry (Biotechnol. Bioeng. 90 (2005) 656-662). In WO 2008/142124 recombinant protein production in Avian EBX^{®} cells is reported.

Yew, N.S., et al., reported about the optimization of plasmid vectors for high-level expression in lung epithelial cells (Hum. Gene Ther. 8 (1997) 575-584).

Winnard, P., et al., reported that robust expression of transgenes in MCF-7 breast cancer cells is expression vector-dependent (BioTechniques 37 (2004) 372).

In WO 2010/097437 a method for producing antibodies is reported.

### Summary of the Invention

It has been found that the performance of an expression vector largely depends on its intended use and that best vectors for transient transfections, stable pools and single clone selection differ.

To highlight the main findings: For transient transfection the bidirectional expression of the antibody light and heavy chain and the use of the full length hCMV promoter including Intron A were advantageous. For stable transfection, however, the in row arrangement of 1) antibody light chain, 2) antibody heavy chain and 3) selection marker has shown to be advantageous.

But whereas the hEF1α promoter is clearly superior to the hCMV promoter in stable pools, the clear opposite effect on single clone level has been found. Here, with the human cytomegalovirus immediate early promoter/enhancer (hCMV) clones with highest productivity were obtained.

Moreover, the hCMV promoter performance can further be improved by combining it with the bGH polyA signal and the terminator sequence of the human gastrin gene (hGT), which increases both productivity and stability of expression.

It has been found that the use of an expression vector comprising an expression cassettes for an antibody heavy chain and an expression cassette for an antibody light chain each comprising a promoter, a structural gene and a polyA signal sequence and optionally a terminator sequence, results in a higher number of antibody producing/secreting cell clones after transfection if i) the promoter is the human cytomegalovirus promoter (hCMV), the polyA signal sequence is the bovine growth hormone polyA signal sequence (bGH polyA) and the terminator sequence is the human gastrin gene transcription terminator sequence (hGT), or 2) the promoter is the human elongation factor 1 alpha promoter (hEF1alpha), the polyA signal sequence is the bovine growth hormone polyA signal sequence (bGH polyA) and the terminator sequence is absent, as disclosed herein.

By using an expression vector as outlined above a higher number of antibody producing/secreting cells can be obtained after transfection and, thus, the required efforts to identify a high producer cell suitable for large scale recombinant antibody production are reduced.

Therefore, the current invention comprises a method for selecting a recombinant stable transfected mammalian cell comprising the following step:
a) transfecting a mammalian cell with an expression vector comprising
   - a first expression cassette comprising in 5' to 3' direction a hCMV promoter, a nucleic acid encoding an antibody light chain, a bGH polyA signal sequence, and a hGT terminator sequence,
   - a second expression cassette comprising in 5' to 3' direction a hCMV promoter, a nucleic acid encoding an antibody heavy chain, a bGH polyA signal sequence, and a hGT terminator sequence, and

   whereby the first expression cassette and the second expression cassette are arranged unidirectional,
   and thereby obtaining a multitude of recombinant mammalian cells,
b) selecting from the multitude of recombinant mammalian cells a (single) recombinant mammalian cell, wherein in cell lines obtained by transfection and selection of stable cell clones the combination of bovine growth hormone polyA signal sequence and human gastrin terminator results in an improved antibody expression yield.

In one embodiment the nucleic acid encoding the antibody light chain and/or the nucleic acid encoding the antibody heavy chain comprises at least one intron.

In one embodiment the nucleic acid encoding the antibody light chain and/or the nucleic acid encoding the antibody heavy chain is cDNA.

In one embodiment the expression plasmid further comprises a selection marker. In one embodiment the expression cassettes and the selection marker are arranged unidirectional. In one embodiment the expression cassettes are arranged in the sequence LC-HC-SM.

In one embodiment the mammalian cell is selected from CHO cell, HEK cell, BHK cell, NS0 cell, and SP2/0 cell. In one embodiment the mammalian cell is a CHO cell for the selection of a stable transfected cell. In one embodiment the mammalian cell is a HEK cell for the selection of a transient transfected cell.

The current invention further comprises a method for producing an antibody comprising the following step:
a) cultivating a mammalian cell comprising
   - a first expression cassette comprising in 5' to 3' direction a hCMV promoter, a nucleic acid encoding an antibody light chain, a bGH polyA signal sequence, and a hGT terminator sequence,
   - a second expression cassette comprising in 5' to 3' direction a hCMV promoter, a nucleic acid encoding an antibody heavy chain, a bGH polyA signal sequence, and a hGT terminator sequence, and
b) recovering the antibody from the cell or the cultivation medium,
whereby the first expression cassette and the second expression cassette are arranged unidirectional for the stable production of the antibody.

In one embodiment the nucleic acid encoding the antibody light chain and/or the nucleic acid encoding the antibody heavy chain comprises at least one intron.

In one embodiment the nucleic acid encoding the antibody light chain and/or the nucleic acid encoding the antibody heavy chain is cDNA.

In one embodiment the expression plasmid further comprises a selection marker. In one embodiment the expression cassettes and the selection marker are arranged unidirectional. In one embodiment the expression cassettes are arranged in the sequence LC-HC-SM.

In one embodiment the mammalian cell is selected from CHO cell, HEK cell, BHK cell, NS0 cell, and SP2/0 cell. In one embodiment the mammalian cell is a CHO cell for the stable production of an antibody. In one embodiment the mammalian cell is a HEK cell for the transient production of an antibody.

The current invention further comprises an expression vector comprising
- a first expression cassette comprising in 5' to 3' direction a hCMV promoter, a nucleic acid encoding an antibody light chain, a bGH polyA signal sequence, and a hGT terminator sequence,
- a second expression cassette comprising in 5' to 3' direction a hCMV promoter, a nucleic acid encoding an antibody heavy chain, a bGH polyA signal sequence, and a hGT terminator sequence.

In one embodiment the nucleic acid encoding the antibody light chain and/or the nucleic acid encoding the antibody heavy chain comprises at least one intron.

In one embodiment the nucleic acid encoding the antibody light chain and/or the nucleic acid encoding the antibody heavy chain is cDNA.

In one embodiment the first expression cassette and the second expression cassette are arranged unidirectional for the selection of a stable transfected cell.

In one embodiment the first expression cassette and the second expression cassette are arranged bidirectional for the selection of a transient transfected cell.

In one embodiment the expression plasmid further comprises a selection marker. In one embodiment the expression cassettes and the selection marker are arranged unidirectional. In one embodiment the expression cassettes are arranged in the sequence LC-HC-SM.

It has been found that for the generation of stable recombinant antibody expressing/secreting cells lines when using a human elongation factor 1 alpha promoter (hEF1alpha) in combination with bGH polyA signal sequence the presence of an hGT terminator sequence reduces obtainable expression yield.

It is disclosed herein that for the stable recombinant production of an antibody the use of an expression vector comprising
- a first expression cassette comprising in 5' to 3' direction a first promoter, a nucleic acid encoding an antibody light chain, a first polyA signal sequence, and optional a first transcription terminator sequence,
- a second expression cassette comprising in 5' to 3' direction a second promoter, a nucleic acid encoding an antibody heavy chain, a second polyA signal sequence, and optional a second transcription terminator sequence, and
- a third expression cassette comprising in 5' to 3' direction a third promoter, a nucleic acid conferring resistance to a selection agent, a third polyA signal sequence, and optional a third transcription terminator sequence,

whereby the three expression cassettes are organized unidirectional and in the sequence first expression cassette-second expression cassette-third expression cassette,
results in an improved expression yield in a stably transfected recombinant mammalian cell.

It is also disclosed herein that in contrast to the above for the transient recombinant production of an antibody the use of an expression vector comprising
- a first expression cassette comprising in 5' to 3' direction a first promoter, a nucleic acid encoding an antibody light chain, a first polyA signal sequence, and optional a first transcription terminator sequence,
- a second expression cassette comprising in 5' to 3' direction a second promoter, a nucleic acid encoding an antibody heavy chain, a second polyA signal sequence, and optional a second transcription terminator sequence, and
- a third expression cassette comprising in 5' to 3' direction a third promoter, a nucleic acid conferring resistance to a selection agent, a third polyA signal sequence, and optional a third transcription terminator sequence,

whereby the expression cassettes are organized bidirectional whereby the first expression cassette and the second expression cassette are arranged in opposite direction,
results in an improved expression yield in a transiently transfected recombinant mammalian cell.

The term in opposite direction denotes that one expression cassette is transcribed in the 5' -> 3' direction and one expression cassette is transcribed in 3'-> 5' direction.

Thus, disclosed herein is the use of an expression vector comprising
- a first expression cassette comprising in 5' to 3' direction a first promoter, a nucleic acid encoding an antibody light chain, a first polyA signal sequence, and optional a first transcription terminator sequence,
- a second expression cassette comprising in 5' to 3' direction a second promoter, a nucleic acid encoding an antibody heavy chain, a second polyA signal sequence, and optional a second transcription terminator sequence, and
- a third expression cassette comprising in 5' to 3' direction a third promoter, a nucleic acid conferring resistance to a selection agent, a third polyA signal sequence, and optional a third transcription terminator sequence,

whereby the three expression cassettes are organized unidirectional and in the sequence first expression cassette-second expression cassette-third expression cassette,
for the stable recombinant production of an antibody in a mammalian cell.

In one embodiment of the invention the expression vector comprises
- a first expression cassette comprising in 5' to 3' direction a first promoter, a nucleic acid encoding an antibody light chain, a first polyA signal sequence, and optional a first transcription terminator sequence,
- a second expression cassette comprising in 5' to 3' direction a second promoter, a nucleic acid encoding an antibody heavy chain, a second polyA signal sequence, and optional a second transcription terminator sequence, and
- a third expression cassette comprising in 5' to 3' direction a third promoter, a nucleic acid conferring resistance to a selection agent, a third polyA signal sequence, and optional a third transcription terminator sequence,
whereby the three expression cassettes are organized unidirectional and in the sequence first expression cassette-second expression cassette-third expression cassette, wherein the first and the second promoter are the hCMV promoter, the first and second polyA signal sequence are the bGH polyA signal sequence, and the transcription termination sequence is present and is the hGT terminator sequence.

Reported herein is the use of an expression plasmid as reported herein for the stable expression of an antibody.

One aspect as reported herein is a eukaryotic cell comprising an expression plasmid as reported herein.

In one embodiment the hCMV promoter has the sequence of SEQ ID NO: 01. This is the hCMV promoter without Intron A and without 5'UTR.

In one embodiment the hCMV promoter has the sequence of SEQ ID NO: 02. This is the hCMV promoter without Intron A and with 5'UTR.

In one embodiment the hCMV promoter has the sequence of SEQ ID NO: 03. This is the full length hCMV promoter with Intron A.

In one embodiment the bovine growth hormone polyA signal sequence has the sequence SEQ ID NO: 09.

In one embodiment the human gastrin terminator has the sequence of SEQ ID NO: 10.

In one embodiment the neomycin selection marker has the sequence of SEQ ID NO: 14.

In one embodiment of all aspects as reported herein the antibody is a bispecific antibody.

In one embodiment the bispecific antibody has a first binding specificity or binding site that specifically binds to a first antigen or a first epitope on an antigen and the bispecific antibody has a second binding specificity or binding site that specifically binds to a second antigen or second epitope on the antigen.

In one embodiment of the invention the expression vector comprises either
- a first expression cassette comprising in 5' to 3' direction a promoter, a nucleic acid encoding a first antibody light chain, a polyA signal sequence, and a terminator sequence,
- a second expression cassette comprising in 5' to 3' direction a promoter, a nucleic acid encoding a second antibody light chain, a polyA signal sequence, and a terminator sequence,
- a third expression cassette comprising in 5' to 3' direction a promoter, a nucleic acid encoding a first antibody heavy chain, a polyA signal sequence, and a terminator sequence,
- a fourth expression cassette comprising in 5' to 3' direction a promoter, a nucleic acid encoding a second antibody heavy chain, a polyA signal sequence, and a terminator sequence,
   or
- a first expression cassette comprising in 5' to 3' direction a promoter, a nucleic acid encoding an antibody light chain, a polyA signal sequence, and a terminator sequence,
- a second expression cassette comprising in 5' to 3' direction a promoter, a nucleic acid encoding a first antibody heavy chain, a polyA signal sequence, and a terminator sequence, and
- a third expression cassette comprising in 5' to 3' direction a promoter, a nucleic acid encoding a second antibody heavy chain, a polyA signal sequence, and a terminator sequence,
whereby the antibody light chain is a common light chain for both antibody heavy chains.

In one embodiment of the invention the expression vector comprises
- an antibody light chain expression cassette,
- a first antibody heavy chain expression cassette,
- a second antibody heavy chain expression cassette, and
- a selection marker expression cassette,

wherein at least one of the antibody heavy chain expression cassettes, the antibody light chain expression cassette, and the selection marker expression cassette are arranged unidirectional, and
wherein the unidirectional expression cassettes are arranged in the 5' to 3' sequence of antibody heavy chain expression cassette, antibody light chain expression cassette and selection marker expression cassette or the unidirectional expression cassettes are arranged in the 5' to 3' sequence of antibody light chain expression cassette, antibody heavy chain expression cassette and selection marker expression cassette.

In one embodiment of the invention the expression vector comprises
- a first antibody light chain expression cassette,
- a second antibody light chain expression cassette,
- a first antibody heavy chain expression cassette,
- a second antibody heavy chain expression cassette, and
- a selection marker expression cassette,

wherein one of the antibody heavy chain expression cassettes, one of the antibody light chain expression cassette, and the selection marker expression cassette are arranged unidirectional, and
wherein the unidirectional expression cassettes are arranged in the 5' to 3' sequence of antibody heavy chain expression cassette, antibody light chain expression cassette and selection marker expression cassette or the unidirectional expression cassettes are arranged in the 5' to 3' sequence of antibody light chain expression cassette, antibody heavy chain expression cassette and selection marker expression cassette.

In one embodiment encodes one of the antibody heavy chain expression cassettes an antibody heavy chain comprising a hole mutation.

In one embodiment encodes one of the antibody heavy chain expression cassettes an antibody heavy chain comprising a knob mutation.

In one embodiment encodes one of the antibody light chain expression cassettes an antibody light chain comprising an antibody light chain variable domain and an antibody heavy chain CH1 domain as constant domain and/or one of the antibody light chain expression cassettes an antibody light chain comprising an antibody light chain variable domain and an antibody light chain CL domain as constant domain.

In one embodiment one of the antibody heavy chain expression cassettes encodes an antibody heavy chain comprising as first constant domain an antibody light chain constant domain (CL), and/or one of the antibody heavy chain expression cassettes encodes an antibody heavy chain comprising as first constant domain an antibody heavy chain CH1 domain.

### Detailed Description of the Invention

### I. General aspects

As known to a person skilled in the art enables the use of recombinant DNA technology the production of numerous derivatives of a nucleic acid and/or polypeptide. Such derivatives can, for example, be modified in one individual or several positions by substitution, alteration, exchange, deletion, or insertion. The modification or derivatization can, for example, be carried out by means of site directed mutagenesis. Such modifications can easily be carried out by a person skilled in the art (see e.g. Sambrook, J., et al., Molecular Cloning: A Laboratory Manual, Cold Spring Harbor Laboratory Press, New York, USA (1999)). The use of recombinant technology enables a person skilled in the art to transform various host cells with heterologous nucleic acid(s). Although the transcription and translation, i.e. expression, machinery of different cells use the same elements, cells belonging to different species may have among other things a different so-called codon usage. Thereby identical polypeptides (with respect to amino acid sequence) may be encoded by different nucleic acid(s). Also, due to the degeneracy of the genetic code, different nucleic acids may encode the same polypeptide.

The use of recombinant DNA technology enables the production of numerous derivatives of a nucleic acid and/or polypeptide. Such derivatives can, for example, be modified in one individual or several positions by substitution, alteration, exchange, deletion, or insertion. The modification or derivatization can, for example, be carried out by means of site directed mutagenesis. Such modifications can easily be carried out by a person skilled in the art (see e.g. Sambrook, J., et al., Molecular Cloning: A Laboratory Manual, Cold Spring Harbor Laboratory Press, New York, USA (1999); Hames, B.D. and Higgins, S.J., Nucleic acid hybridization - a practical approach, IRL Press, Oxford, England (1985)).

The use of recombinant technology enables the transformation of various host cells with heterologous nucleic acid(s). Although the transcription and translation, i.e. expression, machinery of different cells use the same elements, cells belonging to different species may have among other things a different so-called codon usage. Thereby identical polypeptides (with respect to amino acid sequence) may be encoded by different nucleic acid(s). Also, due to the degeneracy of the genetic code, different nucleic acids may encode the same polypeptide.

### DEFINITIONS

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g. scFv); and multispecific antibodies formed from antibody fragments.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgAi, and IgA₂. The heavy chain constant domains that correspond to the different classes of immunoglobulins are called α, δ, ε, γ, and µ, respectively.

The term "expression" as used herein refers to transcription and/or translation processes occurring within a cell. The level of transcription of a nucleic acid sequence of interest in a cell can be determined on the basis of the amount of corresponding mRNA that is present in the cell. For example, mRNA transcribed from a sequence of interest can be quantitated by RT-PCR or by Northern hybridization (see Sambrook et al., 1999, supra). Polypeptides encoded by a nucleic acid of interest can be quantitated by various methods, e.g. by ELISA, by assaying for the biological activity of the polypeptide, or by employing assays that are independent of such activity, such as Western blotting or radioimmunoassay, using immunoglobulins that recognize and bind to the polypeptide (see Sambrook et al., 1999, supra).

An "expression cassette" refers to a construct that contains the necessary regulatory elements, such as promoter and polyadenylation site, for expression of at least the contained nucleic acid in a cell.

An "expression vector" is a nucleic acid providing all required elements for the expression of the comprised structural gene(s) in a host cell. Typically, an expression plasmid comprises a prokaryotic plasmid propagation unit, e.g. for E. coli, comprising an origin of replication, and a selectable marker, an eukaryotic selection marker, and one or more expression cassettes for the expression of the structural gene(s) of interest each comprising a promoter, a structural gene, and a polyadenylation signal (polyA signal sequence). Gene expression is usually placed under the control of a promoter, and such a structural gene is said to be "operably linked to" the promoter. Similarly, a regulatory element and a core promoter are operably linked if the regulatory element modulates the activity of the core promoter.

The term "Fc-region" herein is used to define a C-terminal region of an immunoglobulin heavy chain that contains at least a portion of the constant region. The term includes native sequence Fc-regions and variant Fc-regions. In one embodiment, a human IgG heavy chain Fc-region extends from Cys226, or from Pro230, to the carboxyl-terminus of the heavy chain. However, the C-terminal lysine (Lys447) of the Fc-region may or may not be present. Unless otherwise specified herein, numbering of amino acid residues in the Fc-region or constant region is according to the EU numbering system, also called the EU index, as described in Kabat, E.A., et al., Sequences of Proteins of Immunological Interest, 5th ed., Public Health Service, National Institutes of Health, Bethesda, MD (1991), NIH Publication 91-3242.

An "Fc- region" is a term well known and defined on basis of the papain cleavage of an antibody heavy chain. In one embodiment the Fc-region is either an Fc-region of a human antibody of the subclass IgG4 or an Fc-region of a human antibody of the subclass IgG1, IgG2, or IgG3, which is modified in such a way that no Fcγ receptor (e.g. FcγRIIIa) binding and/or no C1q binding can be detected. In one embodiment the Fc-region is a human Fc-region and especially either from human IgG4 subclass or a mutated Fc-region from human IgG1 subclass. In one embodiment the Fc-region is from human IgG1 subclass with mutations L234A and L235A. While IgG4 shows reduced Fcγ receptor (FcγRIIIa) binding, antibodies of other IgG subclasses show strong binding. However Pro238, Asp265, Asp270, Asn297 (loss of Fc carbohydrate), Pro329, Leu234, Leu235, Gly236, Gly237, Ile253, Ser254, Lys288, Thr307, Gln311, Asn434, or/and His435 are residues which, if altered, provide also reduced Fcγ receptor binding (Shields, R.L., et al., J. Biol. Chem. 276 (2001) 6591-6604; Lund, J., et al., FASEB J. 9 (1995) 115-119; Morgan, A., et al., Immunology 86 (1995) 319-324; EP 0 307 434). In one embodiment the antibody to be expressed in an aspect as reported herein is in regard to Fcγ receptor binding of IgG4 subclass or of IgG1 or IgG2 subclass, with a mutation in L234, L235, and/or D265, and/or contains the PVA236 mutation. In one embodiment the mutations are S228P, L234A, L235A, L235E, and/or PVA236 (PVA236 denotes that the amino acid sequence ELLG (given in one letter amino acid code) from amino acid position 233 to 236 of IgG1 or EFLG of IgG4 is replaced by PVA). In one embodiment the mutations are S228P of IgG4, and L234A and L235A of IgG1. The Fc-region of an antibody is directly involved in ADCC (antibody-dependent cell-mediated cytotoxicity) and CDC (complement-dependent cytotoxicity). A complex which does not bind Fcγ receptor and/or complement factor C1q does not elicit antibody-dependent cellular cytotoxicity (ADCC) and/or complement dependent cytotoxicity (CDC). The knob modification denotes the mutation T366W in the CH3 domain of an antibody (numbering according to Kabat). The hole-modification denotes the mutations T366S, L368A and Y407V in the CH3 domain of an antibody. In addition to the knob and hole modification the mutation S354C in the one CH3 domain and the mutation Y349C in the other CH3 domain can be present.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody", "intact antibody", and "whole antibody" are used herein interchangeably to refer to an antibody having a structure substantially similar to a native antibody structure or having heavy chains that contain an Fc-region as defined herein.

A "gene" denotes a nucleic acid which is a segment e.g. on a chromosome or on a plasmid which can affect the expression of a peptide, polypeptide, or protein. Beside the coding region, i.e. the structural gene, a gene comprises other functional elements e.g. a signal sequence, promoter(s), introns, and/or terminators.

The terms "host cell", "host cell line", and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR" as used herein, refers to each of the regions of an antibody variable domain which are hypervariable in sequence and/or form structurally defined loops ("hypervariable loops"). Generally, native four-chain antibodies comprise six HVRs; three in the VH (HI, H2, H3), and three in the VL (L1, L2, L3). HVRs generally comprise amino acid residues from the hypervariable loops and/or from the "complementarity determining regions" (CDRs), the latter being of highest sequence variability and/or involved in antigen recognition. Exemplary hypervariable loops occur at amino acid residues 26-32 (L1), 50-52 (L2), 91-96 (L3), 26-32 (H1), 53-55 (H2), and 96-101 (H3) (Chothia, C. and Lesk, A.M., J. Mol. Biol. 196 (1987) 901-917). Exemplary CDRs (CDR-L1, CDR-L2, CDR-L3, CDR-H1, CDR-H2, and CDR-H3) occur at amino acid residues 24-34 of L1, 50-56 of L2, 89-97 of L3, 31-35B of H1, 50-65 of H2, and 95-102 of H3 (Kabat, E.A., et al., Sequences of Proteins of Immunological Interest, 5th ed. Public Health Service, National Institutes of Health, Bethesda, MD (1991), NIH Publication 91-3242). With the exception of CDR1 in VH, CDRs generally comprise the amino acid residues that form the hypervariable loops. CDRs also comprise "specificity determining residues," or "SDRs," which are residues that contact antigen. SDRs are contained within regions of the CDRs called abbreviated-CDRs, or a-CDRs. Exemplary a-CDRs (a-CDR-L1, a-CDR-L2, a-CDR-L3, a-CDR-H1, a-CDR-H2, and a-CDR-H3) occur at amino acid residues 31-34 of L1, 50-55 of L2, 89-96 of L3, 31-35B of H1, 50-58 of H2, and 95-102 of H3 (Almagro, J.C. and Fransson, J., Front. Biosci. 13 (2008) 1619-1633). Unless otherwise indicated, HVR residues and other residues in the variable domain (e.g., FR residues) are numbered herein according to Kabat et al., supra.

An "internal ribosome entry site" or "IRES" describes a sequence which functionally promotes translation initiation independent from the gene 5' of the IRES and allows two cistrons (open reading frames) to be translated from a single transcript in an animal cell. The IRES provides an independent ribosome entry site for translation of the open reading frame immediately downstream (downstream is used interchangeably herein with 3') of it. Unlike bacterial mRNA which can be polycistronic, i.e. encode several different polypeptides that are translated sequentially from the mRNAs, most mRNAs of animal cells are monocistronic and code for the synthesis of only one protein. With a polycistronic transcript in a eukaryotic cell, translation would initiate from the 5' most translation initiation site, terminate at the first stop codon, and the transcript would be released from the ribosome, resulting in the translation of only the first encoded polypeptide in the mRNA. In a eukaryotic cell, a polycistronic transcript having an IRES operably linked to the second or subsequent open reading frame in the transcript allows the sequential translation of that downstream open reading frame to produce the two or more polypeptides encoded by the same transcript. The use of IRES elements in vector construction has been previously described, see, e.g., Pelletier, J., et al., Nature 334 (1988) 320-325; Jang, S.K., et al., J. Virol. 63 (1989) 1651-1660; Davies, M.V., et al., J. Virol. 66 (1992) 1924-1932; Adam, M.A., et al., J. Virol. 65 (1991) 4985-4990; Morgan, R.A., et al. Nucl. Acids Res. 20 (1992) 1293-1299; Sugimoto, Y, et al., Biotechnology 12 (1994) 694-698; Ramesh, N., et al., Nucl. Acids Res. 24 (1996) 2697-2700; and Mosser, D.D., et al., BioTechniques 22 (1997) 150-152).

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

A "nucleic acid" as used herein, refers to a polymeric molecule consisting of individual nucleotides (also called bases) a, c, g, and t (or u in RNA), for example to DNA, RNA, or modifications thereof. This polynucleotide molecule can be a naturally occurring polynucleotide molecule or a synthetic polynucleotide molecule or a combination of one or more naturally occurring polynucleotide molecules with one or more synthetic polynucleotide molecules. Also encompassed by this definition are naturally occurring polynucleotide molecules in which one or more nucleotides are changed (e.g. by mutagenesis), deleted, or added. A nucleic acid can either be isolated, or integrated in another nucleic acid, e.g. in an expression cassette, a plasmid, or the chromosome of a host cell. A nucleic acid is likewise characterized by its nucleic acid sequence consisting of individual nucleotides.

To a person skilled in the art procedures and methods are well known to convert an amino acid sequence, e.g. of a polypeptide, into a corresponding nucleic acid sequence encoding this amino acid sequence. Therefore, a nucleic acid is characterized by its nucleic acid sequence consisting of individual nucleotides and likewise by the amino acid sequence of a polypeptide encoded thereby.

A "nucleic acid" as used herein, refers to a naturally occurring or partially or fully non-naturally occurring nucleic acid encoding a polypeptide which can be produced recombinantly. The nucleic acid can be build up of DNA-fragments which are either isolated or synthesized by chemical means. The nucleic acid can be integrated into another nucleic acid, e.g. in an expression plasmid or the genome/chromosome of a eukaryotic host cell. Plasmid includes shuttle and expression plasmids. Typically, the plasmid will also comprise a prokaryotic propagation unit comprising an origin of replication (e.g. the ColE1 origin of replication) and a selectable marker (e.g. ampicillin or tetracycline resistance gene), for replication and selection, respectively, of the plasmid in prokaryotes.

"Operably linked" refers to a juxtaposition of two or more components, wherein the components so described are in a relationship permitting them to function in their intended manner. For example, a promoter and/or enhancer are operably linked to a coding sequence, if it acts in cis to control or modulate transcription of the linked sequence. Generally, but not necessarily, the DNA sequences that are "operably linked" are contiguous and, where necessary to join two protein encoding regions such as a secretory leader and a polypeptide, contiguous and in (reading) frame. However, although an operably linked promoter is generally located upstream of the coding sequence, it is not necessarily contiguous with it. Enhancers do not have to be contiguous. An enhancer is operably linked to a coding sequence if the enhancer increases transcription of the coding sequence. Operably linked enhancers can be located upstream, within or downstream of coding sequences and at considerable distance from the promoter. A polyadenylation site is operably linked to a coding sequence if it is located at the downstream end of the coding sequence such that transcription proceeds through the coding sequence into the polyadenylation sequence. A translation stop codon is operably linked to an exonic nucleic acid sequence if it is located at the downstream end (3' end) of the coding sequence such that translation proceeds through the coding sequence to the stop codon and is terminated there. Linking is accomplished by recombinant methods known in the art, e.g., using PCR methodology and/or by ligation at convenient restriction sites. If convenient restriction sites do not exist, then synthetic oligonucleotide adaptors or linkers are used in accord with conventional practice.

A "polycistronic transcription unit" is a transcription unit in which more than one structural gene is under the control of the same promoter.

The term "polyadenylation signal" as used within this application denotes a nucleic acid sequence used to induce cleavage and polyadenylation of primary transcripts of a specific nucleic acid sequence segment. The 3' untranslated region comprising a polyadenylation signal can be selected from the group consisting of the 3' untranslated region comprising a polyadenylation signals derived from SV40, the gene for bovine growth hormone (bGH), immunoglobulin genes, and the thymidine kinase gene (tk, e.g. Herpes Simplex thymidine kinase polyadenylation signal).

A "promoter" refers to a polynucleotide sequence that controls transcription of a gene/structural gene or nucleic acid sequence to which it is operably linked. A promoter includes signals for RNA polymerase binding and transcription initiation. The promoters used will be functional in the cell type of the host cell in which expression of the selected sequence is contemplated. A large number of promoters including constitutive, inducible and repressible promoters from a variety of different sources, are well known in the art (and identified in databases such as GenBank) and are available as or within cloned polynucleotides (from, e.g., depositories such as ATCC as well as other commercial or individual sources).

A "promoter" comprises a nucleotide sequence that directs the transcription of a structural gene. Typically, a promoter is located in the 5' non-coding or untranslated region of a gene, proximal to the transcriptional start site of a structural gene. Sequence elements within promoters that function in the initiation of transcription are often characterized by consensus nucleotide sequences. These promoter elements include RNA polymerase binding sites, TATA sequences, CAAT sequences, differentiation-specific elements (DSEs; McGehee, R.E., et al., Mol. Endocrinol. 7 (1993) 551), cyclic AMP response elements (CREs), serum response elements (SREs; Treisman, R., Seminars in Cancer Biol. 1 (1990) 47), glucocorticoid response elements (GREs), and binding sites for other transcription factors, such as CRE/ATF (O'Reilly, M.A., et al., J. Biol. Chem. 267 (1992) 19938), AP2 (Ye, J., et al., J. Biol. Chem. 269 (1994) 25728), SP1, cAMP response element binding protein (CREB; Loeken, M.R., Gene Expr. 3 (1993) 253) and octamer factors (see, in general, Watson, et al., (eds.), Molecular Biology of the Gene, 4th ed. (The Benjamin/Cummings Publishing Company, Inc. (1987)), and Lemaigre, F.P. and Rousseau, G.G., Biochem. J. 303 (1994) 1-14). If a promoter is an inducible promoter, then the rate of transcription increases in response to an inducing agent. In contrast, the rate of transcription is not regulated by an inducing agent if the promoter is a constitutive promoter. Repressible promoters are also known. For example, the c-fos promoter is specifically activated upon binding of growth hormone to its receptor on the cell surface. Tetracycline (tet) regulated expression can be achieved by artificial hybrid promoters that consist e.g. of a CMV promoter followed by two Tet-operator sites. The Tet-repressor binds to the two Tet-operator sites and blocks transcription. Upon addition of the inducer tetracycline, Tet-repressor is released from the Tet-operator sites and transcription proceeds (Gossen, M. and Bujard, H., PNAS 89 (1992) 5547-5551). For other inducible promoters including metallothionein and heat shock promoters, see, e.g., Sambrook et al. (supra) and Gossen et al., Curr. Opin. Biotech. 5 (1994) 516-520. Among the eukaryotic promoters that have been identified as strong promoters for high-level expression are the SV40 early promoter, adenovirus major late promoter, mouse metallothionein-I promoter, Rous sarcoma virus long terminal repeat, Chinese hamster elongation factor 1 alpha (CHEF-1, see e.g. US 5,888,809), human EF-1 alpha, ubiquitin, and human cytomegalovirus immediate early promoter (CMV IE).

The "promoter" can be constitutive or inducible. An enhancer (i.e., a cis-acting DNA element that acts on a promoter to increase transcription) may be necessary to function in conjunction with the promoter to increase the level of expression obtained with a promoter alone, and may be included as a transcriptional regulatory element. Often, the polynucleotide segment containing the promoter will include enhancer sequences as well (e.g., CMV or SV40).

The terms "stably transformed", "stable transfected", or "stable" as used within this application denotes a heritable and stable integration of exogenous nucleic acid into a host cell genome/chromosome. A stable transfected cell is obtained after a cell selection process under selective growth conditions, i.e. in the presence of one or more selection markers.

A "structural gene" denotes the region of a gene without a signal sequence, i.e. the coding region.

The term "transcription terminator" denotes a DNA sequence of 50-750 base pairs in length which gives the RNA polymerase the signal for termination of the mRNA synthesis. Very efficient (strong) terminators at the 3' end of an expression cassette are advisable to prevent the RNA polymerase from reading through particularly when using strong promoters. Inefficient transcription terminators can lead to the formation of an operon-like mRNA which can be the reason for an undesired, e.g. plasmid-coded, gene expression.

Within the scope of the present invention, transfected cells may be obtained with substantially any kind of transfection method known in the art. For example, the nucleic acid may be introduced into the cells by means of electroporation or microinjection. Alternatively, lipofection reagents such as FuGENE 6 (Roche Diagnostics GmbH, Germany), X-tremeGENE (Roche Diagnostics GmbH, Germany), and LipofectAmine (Invitrogen Corp., USA) may be used. Still alternatively, the nucleic acid may be introduced into the cell by appropriate viral vector systems based on retroviruses, lentiviruses, adenoviruses, or adeno-associated viruses (Singer, O., Proc. Natl. Acad. Sci. USA 101 (2004) 5313-5314).

The term "transient transfection" as used within this application denotes a process in which the nucleic acid introduced into a cell does not integrate into the genome or chromosomal DNA of that cell. It is in fact maintained as an extrachromosomal element, e.g. as an episome, in the cell. Transcription processes of the nucleic acid of the episome are not affected and e.g. a protein encoded by the nucleic acid of the episome is produced. A transient transfection results in a "transient transfected" cell.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs) (see, e.g., Kindt, T.J., et al., Kuby Immunology, 6th ed., W.H. Freeman and Co., N.Y. (2007), page 91). A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively (see, e.g., Portolano, S., et al., J. Immunol. 150 (1993) 880-887; Clackson, T., et al., Nature 352 (1991) 624-628).

The term "vector" as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

### ANTIBODY

The methods and compositions provided herein are for the production of recombinant monoclonal antibodies. An antibody can be of various structures, such as but not limited to monospecific antibodies, multispecific antibodies (e.g., bispecific antibodies), antibody fragments, monovalent antibodies, multivalent antibodies (e.g. bivalent antibodies).

In certain embodiments, the antibody is an antibody fragment. Antibody fragments include, but are not limited to, Fab, Fab', Fab'-SH, F(ab')₂, Fv, and scFv fragments, and other fragments described below. For a review of certain antibody fragments, see Hudson, P.J., et al., Nat. Med. 9 (2003) 129-134. For a review of scFv fragments, see, e.g., Plueckthun, A., In: The Pharmacology of Monoclonal Antibodies, Vol. 113, Rosenburg and Moore (eds.), Springer-Verlag, New York (1994), pp. 269-315; see also WO 1993/16185; and US 5,571,894 and US 5,587,458. For discussion of Fab and F(ab')₂ fragments comprising salvage receptor binding epitope residues and having increased in vivo half-life, see US 5,869,046.

Diabodies are antibody fragments with two antigen-binding sites that may be bivalent or bispecific (see, for example, EP 0 404 097; WO 1993/01161; Hudson, P.J., et al., Nat. Med. 9 (2003) 129-134; and Holliger, P., et al., Proc. Natl. Acad. Sci. USA 90 (1993) 6444-6448). Triabodies and tetrabodies are also described in Hudson, P.J., et al., Nat. Med. 9 (2003) 129-134).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see, e.g., US 6,248,516).

Antibody fragments can be made by various techniques, including but not limited to proteolytic digestion of an intact antibody as well as production by recombinant host cells (e.g. E. coli or phage), as described herein.

In certain embodiments, the antibody is a chimeric antibody. Certain chimeric antibodies are described, e.g., in US 4,816,567; and Morrison, S.L., et al., Proc. Natl. Acad. Sci. USA 81 (1984) 6851-6855. In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro, J.C. and Fransson, J., Front. Biosci. 13 (2008) 1619-1633, and are further described, e.g., in Riechmann, I., et al., Nature 332 (1988) 323-329; Queen, C., et al., Proc. Natl. Acad. Sci. USA 86 (1989) 10029-10033; US 5,821,337, US 7,527,791, US 6,982,321, and US 7,087,409; Kashmiri, S.V., et al., Methods 36 (2005) 25-34 (describing SDR (a-CDR) grafting); Padlan, E.A., Mol. Immunol. 28 (1991) 489-498 (describing "resurfacing"); Dall'Acqua, W.F., et al., Methods 36 (2005) 43-60 (describing "FR shuffling"); and Osbourn, J., et al., Methods 36 (2005) 61-68 and Klimka, A., et al., Br. J. Cancer 83 (2000) 252-260 (describing the "guided selection" approach to FR shuffling).

Human framework regions that may be used for humanization include but are not limited to: framework regions selected using the "best-fit" method (see, e.g., Sims, M.J., et al., J. Immunol. 151 (1993) 2296-2308); framework regions derived from the consensus sequence of human antibodies of a particular subgroup of light or heavy chain variable regions (see, e.g., Carter, P., et al., Proc. Natl. Acad. Sci. USA 89 (1992) 4285-4289; and Presta, L.G., et al., J. Immunol. 151 (1993) 2623-2632); human mature (somatically mutated) framework regions or human germline framework regions (see, e.g., Almagro, J.C. and Fransson, J., Front. Biosci. 13 (2008) 1619-1633); and framework regions derived from screening FR libraries (see, e.g., Baca, M., et al., J. Biol. Chem. 272 (1997) 10678-10684 and Rosok, M.J., et al., J. Biol. Chem. 271 (19969 22611-22618).

In certain embodiments, the antibody is a human antibody. Human antibodies can be produced using various techniques known in the art. Human antibodies are described generally in van Dijk, M.A. and van de Winkel, J.G., Curr. Opin. Pharmacol. 5 (2001) 368-374 and Lonberg, N., Curr. Opin. Immunol. 20 (2008) 450-459.

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, N., Nat. Biotech. 23 (2005) 1117-1125 and also, e.g., US 6,075,181 and US 6,150,584 describing XENOMOUSE^{™} technology; US 5,770,429 describing HUMAB^{®} technology; US 7,041,870 describing K-M MOUSE^{®} technology, and US 2007/0061900, describing VELOCIMOUSE^{®} technology. Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described (see, e.g., Kozbor, D., J. Immunol. 133 (1984) 3001-3005; Brodeur, B.R., et al., Monoclonal Antibody Production Techniques and Applications, Marcel Dekker, Inc., New York (1987), pp. 51-63; and Boerner, P., et al., J. Immunol. 147 (1991) 86-95). Human antibodies generated via human B-cell hybridoma technology are also described in Li, J., et al., Proc. Natl. Acad. Sci. USA 103 (2006) 3557-3562. Additional methods include those described, for example, in US 7,189,826 (describing production of monoclonal human IgM antibodies from hybridoma cell lines) and Ni, J., Xiandai Mianyixue 26 (2006) 265-268 (describing human-human hybridomas). Human hybridoma technology (Trioma technology) is also described in Vollmers, H.P. and Brandlein, S., Histology and Histopathology 20 (2005) 927-937 and Vollmers, H.P. and Brandlein, S., Methods and Findings in Experimental and Clinical Pharmacology 27 (2005) 185-191.

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

Antibodies may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom, H.R., et al., Methods in Molecular Biology 178 (2001) 1-37 and further described, e.g., in the McCafferty, J., et al., Nature 348 (1990) 552-554; Clackson, T., et al., Nature 352 (1991) 624-628; Marks, J.D., et al., J. Mol. Biol. 222 (1992) 581-597; Marks, J.D. and Bradbury, A., Methods in Molecular Biology 248 (2003) 161-175; Sidhu, S.S., et al., J. Mol. Biol. 338 (2004) 299-310; Lee, C.V., et al., J. Mol. Biol. 340 (2004) 1073-1093; Fellouse, F.A., Proc. Natl. Acad. Sci. USA 101 (2004) 12467-12472; and Lee, C.V., et al., J. Immunol. Methods 284 (2004) 119-132.

In certain phage display methods, repertoires of VH and VL genes are separately cloned by polymerase chain reaction (PCR) and recombined randomly in phage libraries, which can then be screened for antigen-binding phage as described in Winter, G., et al., Ann. Rev. Immunol. 12 (1994) 433-455. Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self-antigens without any immunization as described by Griffiths, A.D., et al., EMBO J. 12 (1993) 725-734. Finally, naive libraries can also be made synthetically by cloning non-rearranged V-gene segments from stem cells, and using PCR primers containing random sequence to encode the highly variable CDR3 regions and to accomplish rearrangement in vitro, as described by Hoogenboom, H.R. and Winter, G., J. Mol. Biol. 227 (1992) 381-388. Patent publications describing human antibody phage libraries include, for example, US 5,750,373, and US 2005/0079574, US 2005/0119455, US 2005/0266000, US 2007/0117126, US 2007/0160598, US 2007/0237764, US 2007/0292936, and US 2009/0002360.

Antibodies or antibody fragments isolated from human antibody libraries are considered human antibodies or human antibody fragments herein.

In certain embodiments, the antibody is a multispecific antibody, e.g. a bispecific antibody. Multispecific antibodies are monoclonal antibodies that have binding specificities for at least two different sites. In certain embodiments, one of the binding specificities is for a first antigen and the other is for a different second antigen. In certain embodiments, bispecific antibodies may bind to two different epitopes of the same antigen. Bispecific antibodies may also be used to localize cytotoxic agents to cells which express the antigen. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein, C. and Cuello, A.C., Nature 305 (1983) 537-540, WO 93/08829, and Traunecker, A., et al., EMBO J. 10 (1991) 3655-3659), and "knob-in-hole" engineering (see, e.g., US 5,731,168). Multi-specific antibodies may also be made by engineering electrostatic steering effects for making antibody Fc-heterodimeric molecules (WO 2009/089004); cross-linking two or more antibodies or fragments (see, e.g., US 4,676,980, and Brennan, M., et al., Science 229 (1985) 81-83); using leucine zippers to produce bi-specific antibodies (see, e.g., Kostelny, S.A., et al., J. Immunol. 148 (1992) 1547-1553; using "diabody" technology for making bispecific antibody fragments (see, e.g., Holliger, P., et al., Proc. Natl. Acad. Sci. USA 90 (1993) 6444-6448); and using single-chain Fv (sFv) dimers (see, e.g., Gruber, M., et al., J. Immunol. 152 (1994) 5368-5374); and preparing trispecific antibodies as described, e.g., in Tutt, A., et al., J. Immunol. 147 (1991) 60-69).

Engineered antibodies with three or more functional antigen binding sites, including "Octopus antibodies", are also included herein (see, e.g. US 2006/0025576).

The antibody can be a "Dual Acting Fab" or "DAF" comprising an antigen binding site that binds to a first antigen as well as another, different antigen (see, US 2008/0069820, for example).

The antibody or fragment can also be a multispecific antibody as described in WO 2009/080251, WO 2009/080252, WO 2009/080253, WO 2009/080254, WO 2010/112193, WO 2010/115589, WO 2010/136172, WO 2010/145792, or WO 2010/145793.

### METHODS

Where the antibody comprises an Fc-region, the carbohydrate attached thereto may be altered. Native antibodies produced by mammalian cells typically comprise a branched, biantennary oligosaccharide that is generally attached by an N-linkage to Asn297 of the CH2 domain of the Fc-region (see, e.g., Wright, A. and Morrison, S.L., TIBTECH 15 (1997) 26-32). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure.

The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc-region (EU numbering according to Kabat of Fc-region residues); however, Asn297 may also be located about ± 3 amino acids upstream or downstream of position 297, i.e., between positions 294 and 300, due to minor sequence variations in antibodies. Such fucosylation variants may have improved ADCC function (see, e.g., US 2003/0157108; US 2004/0093621). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki, A., et al., J. Mol. Biol. 336 (2004) 1239-1249; Yamane-Ohnuki, N., et al., Biotech. Bioeng. 87 (2004) 614-622. Examples of cell lines capable of producing defucosylated antibodies include Lec13 CHO cells deficient in protein fucosylation (Ripka, J., et al., Arch. Biochem. Biophys. 249 (1986) 533-545; US 2003/0157108; and WO 2004/056312, especially at Example 11), and knockout cell lines, such as alpha-1,6-fucosyltransferase gene, FUT8, knockout CHO cells (see, e.g., Yamane-Ohnuki, N., et al., Biotech. Bioeng. 87 (2004) 614-622; Kanda, Y., et al., Biotechnol. Bioeng. 94 (2006) 680-688; and WO 2003/085107).

Antibodies with bisected oligosaccharides, e.g., in which a biantennary oligosaccharide attached to the Fc-region of the antibody is bisected by GlcNAc, may have reduced fucosylation and/or improved ADCC function. Examples of such antibody variants are described, e.g., in WO 2003/011878; US 6,602,684; and US 2005/0123546. Antibody variants with at least one galactose residue in the oligosaccharide attached to the Fc-region can also be produced. Such antibody variants may have improved CDC function. Such antibody variants are described, e.g., in WO 1997/30087; WO 1998/58964; and WO 1999/22764.

Antibodies may be produced using recombinant methods and compositions, e.g., as described in US 4,816,567. Nucleic acid may encode an amino acid sequence comprising the VL and/or an amino acid sequence comprising the VH of the antibody (e.g., the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell (e.g., Y0, NS0, Sp2/0). In one embodiment, a method of making an antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an antibody, nucleic acid encoding an antibody is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc-region effector function are not needed. For expression of antibody fragments and polypeptides in bacteria, see, e.g., US 5,648,237, US 5,789,199, and US 5,840,523; see also Charlton, K.A., In: Methods in Molecular Biology, Vol. 248, Lo, B.K.C. (ed.), Humana Press, Totowa, NJ (2003), pp. 245-254, describing expression of antibody fragments in E. coli. After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern (see Gerngross, T.U., Nat. Biotech. 22 (2004) 1409-1414; and Li, H., et al., Nat. Biotech. 24 (2006) 210-215).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells.

Plant cell cultures can also be utilized as hosts (see, e.g., US 5,959,177, US 6,040,498, US 6,420,548, US 7,125,978, and US 6,417,429 (describing PLANTIBODIES^{™} technology for producing antibodies in transgenic plants)).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham, F.L., et al., J. Gen Virol. 36 (1977) 59-74); baby hamster kidney cells (BHK); mouse sertoli cells (TM4 cells as described, e.g., in Mather, J.P., Biol. Reprod. 23 (1980) 243-252); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather, J.P., et al., Annals N.Y. Acad. Sci. 383 (1982) 44-68; MRC 5 cells; and FS4 cells. Other useful mammalian host cell lines include Chinese hamster ovary (CHO) cells, including DHFR negative (DHFR-) CHO cells (Urlaub, G., et al., Proc. Natl. Acad. Sci. USA 77 (1980) 4216-4220); and myeloma cell lines such as Y0, NS0 and Sp2/0. For a review of certain mammalian host cell lines suitable for antibody production, see, e.g., Yazaki, P. and Wu, A.M., Methods in Molecular Biology, Vol. 248, Lo, B.K.C. (ed.), Humana Press, Totowa, NJ (2004), pp. 255-268.

### II. Specific aspects of the invention

It has been found that depending on the vector organization the performance of the expression vector differs for (1) the vector design, and (2) identical vectors between transient and stable transfections.

It has been found that the optimal vector organization for transient and stable transfections can clearly differ. Without being bound by this theory several points might contribute to these differences: 1) transcriptional interference phenomena between integrated vector copies in the host genome which depend on and are specific for the respective vector design and that do not exist in the transient system, 2) the influence of the selection process and selection stringency which depends on the respective vector organization and that plays an important role in in the stable but not in the transient system, and 3) the optimal LC to HC polypeptide ratio which obviously differs for transient and stable expression of monoclonal antibodies and that is lower in transient than in stable transfections.

It has been found that for transient transfections the bidirectional expression of the LC and HC reached highest product titer of all tested vector organizations. Without being bound by theory it is assumed that this vector design fulfill both criteria for an optimal IgG expression A) high expression levels of the LC and HC polypeptides and B) an optimal 'transient' LC to HC polypeptide ratio, and C) the bidirectional expression of the LC and HC might also minimize transcriptional interference mechanisms as read through effects of the RNA polymerase

It has been found that for stable transfections, however, the bidirectional expression of LC and HC was worse than the in row organization LC-HC-SM. Without being bound by this theory 1) the convergent organization of the expression cassettes for LC, HC and SM might reduce transcriptional interference phenomena between integrated vector copies, 2) the LC upstream of the HC obviously facilitates a LC to HC polypeptide ratio that is (more) optimal for stable transfections, and 3) the downstream position of the selection marker obviously increases stringency of selection. Moreover, the percentage of IgG producing cells and productivity of cell lines has been found to be increased. Increasing the concentration of selection pressure for vectors containing the selection marker bidirectionally upstream of the antibody expression cassettes did not increase productivity of stable pools or clones although stringency of selection clearly increased (data not shown).

It has been found that the concomitant exchange of the hCMV promoter and the SV40 poly signal by the hEF1α promoter and the bGH polyA signal significantly increased transient product titers when the antibody expression cassettes were unidirectionally organized but decreased product titers when the LC and HC expression cassettes were positioned bidirectionally.

It has been found that the hEF1α promoter generates a high number of well-producing and a very low number of non- or low-producing clones. However, product titers for the best individual clones of the hEF1α promoter in fed-batch analysis were lower than for clones of the hCMV promoter. But the overall number of top clones for the hCMV promoter is relatively low and their identification usually requires high screening efforts.

It has been found that the use of hGT significantly increased productivity for vectors containing the hCMV promoter when combined with the SV40 or bGH polyA signal in transient but also in stable transfections. For vectors containing the hEf1α promoter, however, its effect on product titer was negligible when combined with the bGH polyA signal. Thus, it has been found that the influence of the hGT on the vector performance is dependent on the used promoter.

The choice of appropriate clones for the final evaluation in fully controlled large scale fermentations is usually based on batch or fed batch analysis in shake flasks. It has been found that differences in the performance and ranking of some expression vectors or elements between batch and fed-batch analysis are present. Replacing the SV40 polyA by the bGH polyA signal increased productivity for vectors containing the hCMV promoter in batch but not in fed-batch analysis. The hGT has no significant influence on product titer of clones in batch but in fed-batch analysis. Differences between vectors differing in the position of selection marker or in the promoter (hEf1α or hCMV promoter) were moderately pronounced in batch- but strongly evident in fed-batch analysis. Expression levels and specific production rates are higher in a fed-batch than in a batch mode.

A good correlation in the performance of fed-batch analysis and 2L fermentations for most clones has been found, not only on level of absolute product titers, but also in ranking between different vectors and clones.

It has been found that the downstream position of the selection marker slightly reduced loss in productivity compared to the bidirectional position of the selection marker upstream of the antibody expression cassettes. Without being bound by theory, this might be due to increased selection stringency and thus a higher mRNA level or by an improved LC to HC mRNA or polypeptide ratio. Both factors might lead to a higher tolerance towards changes in productivity.

The bGH polyA signal significantly decreased stability of antibody expression in clones compared to the SV40 polyA signal. However, the insertion of the hGT downstream of the bGH polyA signal clearly increased stability of expression. The positive effect of the hGT on stability was most apparent in absence of selection pressure.

Stability analysis of stable pools revealed that cells rapidly lost productivity when generated with the hCMV but not when generated with the hEf1α promoter. Strikingly, although the hGT decreased productivity of clones for vectors containing the hEf1α promoter it slightly increased their stability.

Only a small portion of the clones significantly produces antibody after the selection process. However, modifications in vector organization and/or elements significantly increased the ratio of IgG producing to non-producing clones. Different vector organizations and thus different expression levels of the selection marker that determine stringency of selection also clearly affect the percentage of IgG producing cells. Statistical simulations based on data of the screening process demonstrated that some expression vectors also bear the potential to decrease workload during screening process considerably. This fact has a major impact on costs for biopharmaceutical companies.

### IMPROVEMENT OF THE TRANSCRIPTION PROCESS

### Transcription initiation by the promoter

The promoter determines the transcription level of a gene and therefore has a strong influence on productivity and stability of cell lines:
- transcription initiation mediated by the promoter determines the amount of mRNA that can be translated into recombinant protein;
- despite stable integration long-term productivity of clones can decrease rapidly (as a result of genetic gene silencing (for example by promoter methylation));
- promoter activity is cell type dependent.

Promoters shown to be strong promoters in a variety of cell lines are described. It is known that introns can contain enhancer-like elements. Often, the first intron (Intron A) contains most of the regulatory elements. This Intron A is the first occurring intron within the full length natural promoter sequence/organization.

### Polyadenylation of the mRNA mediated by the polyA signal

Polyadenylation of the mRNA has various functions. It strongly influences the mRNA export out of the nucleus, the translational initiation and the mRNA stability. The efficiency of the polyadenylation process therefore has a strong influence on the expression level of a gene and, thus, on productivity.

Several publications have shown that the polyadenylation signal can have a strong influence on protein expression. It has been reported that the polyA signal derived by the bovine growth hormone (bGH polyA signal) can result in enhanced protein expression when substituted for the SV40 polyA signal.

It has been found that the bovine growth hormone polyA signal can have improved properties in the recombinant production of antibody chains.

### Transcription termination supported by a transcription terminator

The elements comprised in an expression vector can strongly interfere with each other (transcriptional interference). This is due to competition during the transcription process. For example, suboptimal promoter accession for transcription factors and/or the RNA polymerase due to steric constraints and reduced availability of resources of the transcription machinery. Interference can also occur between different close neighboring expression cassettes. For example, a read through of the RNA polymerase from a first through a second expression unit due to an inefficient transcription termination can occur.

It has been reported that an inefficient transcription termination may lead to transcriptional interference, which can result in inefficient expression of a gene. The use of the human gastrin gene transcription terminator (hGT) can improve transcription termination and therefore contribute to an enhanced expression of recombinant proteins. This effect depends on the promoter with which the hGT is combined. Further effective transcription terminators are known.

It has been found that by the insertion of the sequence of the human gastrin transcription terminator after the early SV40 polyA signal of antibody expression cassettes an enhanced transcription termination and prevention of transcriptional interference between expression cassettes e.g. of an antibody light chain and an antibody heavy chain, can be achieved.

### RESULTS

Herein are reported expression vectors for enhanced expression of one or more coding nucleic acids, e.g. of structural genes encoding antibody chains.

It has been found that the productivity of a recombinant cell expressing the structural gene is improved when the genetic elements required for transcription are chosen and arranged properly.

### - polyA signal sequence and transcription terminator sequence

In vector p5068 the expression cassettes were terminated by the SV40 polyA signal.

In vector px6001 the expression cassettes additionally contained the human gastrin transcription terminator (hGT) that was placed downstream of the SV40 PolyA signal.

In vector px6007 the expression cassettes were terminated by the bGH polyA signal and the hGT transcription terminator.

In vector px6008 the expression cassettes were terminated by the bGH polyA signal without an additional transcription terminator.

These vectors were transiently transfected into CHO-K1 cells and six days after transfection cell culture supernatants were harvested and the amount of produced antibody in the cultivation supernatant was determined by ELISA.

The replacement of the SV40 polyA signal by the bGH polyA signal and the addition/insertion of the human gastrin terminator (hGT) after the SV40 polyA signal (vector px6001) resulted in an increase in productivity in transient expression of about 45 % to 30 % compared to the control vector p5068. The combination of the bGH polyA signal and the human gastrin terminator (hGT) resulted in biggest titer increase (+ 58 %) compared to the control vector p5068.

| **Vector** | **amount of antibody in the supernatant [µg/ml]** | **vector elements** |
|---|---|---|
| p5068 | 4.6 | SV40 polyA only |
| px6001 | 6.1 | SV40 polyA and hGT terminator |
| px6007 | 7.4 | bGH polyA + hGT terminator |
| px6008 | 6.8 | bGH polyA only |

Thus, it has been found that in transient expression systems a polyadenylation by the bGH polyA signal and an improved transcription termination by the addition of the human gastrin transcription terminator enhances antibody secretion.

The vectors p5068, px6001, px6007 and px6008 were used to generate stable antibody expressing cell lines.

| **vector** | **promoter** | **polyA signal** | **transcription terminator** |
|---|---|---|---|
| p5068 | short human CMV promoter without Intron A | SV40 polyA signal | no transcription terminator |
| px6001 | short human CMV promoter without Intron A | SV40 polyA signal | human gastrin transcription terminator (hGT) |
| px6007 | short human CMV promoter without Intron A | bGH polyA signal | human gastrin transcription terminator (hGT) |
| px6008 | short human CMV promoter without Intron A | bGH polyA signal | no transcription terminator |

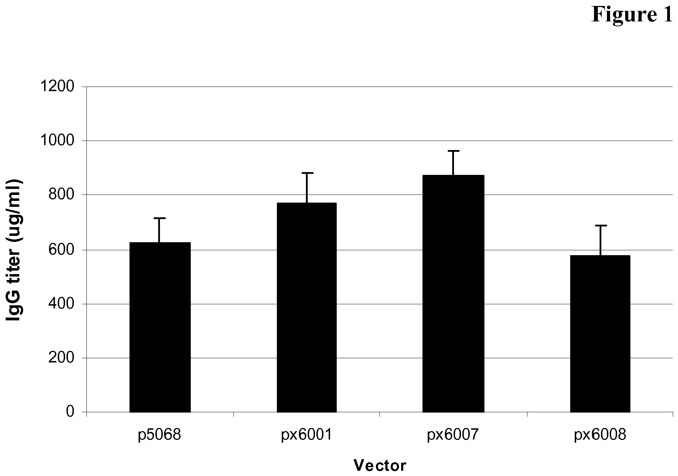

The average productivity of the best 15 single clones generated with the vector p5068 in batch antibody production was 624 µg/ml. Clones generated with the vector px6001 (containing the human gastrin terminator (hGT)) or the vector px6007 (containing a combination of the bGH polyA signal and the hGT) had a productivity that was increased by 23 % and 40 %, respectively (770 µg/ml for vector px6001 and 872 µg/ml for vector px6007) compared to vector p5068 (see Figure 1). This increase in productivity is also reflected on top clone level (+ 23 % for vector px6001 (939 µg/ml) and + 31 % for vector px6007 (1001 µg/ml); values were calculated for the three best clones obtained with each vector).

Clones generated with the vector px6008 had an average productivity of the 15 best clones of 576 µg/ml.

The average productivity of the three best clones for the vector px6008 is 760 µg/ml.

### - promoter sequence

In vector px6051 the expression cassettes comprised the full length human CMV promoter with Intron A and a SV40 polyA signal.

In vector px6052 the expression cassettes comprised the human Ef1α promoter and a SV40 polyA signal.

In vector px6062 the expression cassettes comprised the full length human CMV promoter with Intron A and the bGH polyA signal and the human gastrin terminator.

In vector px6063 the expression cassettes comprised the human Ef1α promoter and the bGH polyA signal and the human gastrin terminator.

| **vector** | **promoter** | **polyA signal** | **transcription terminator** |
|---|---|---|---|
| px6051 | full length hCMV with Intron A | SV40 polyA signal | no transcription terminator |
| px6052 | hEf1α promoter with Intron A | SV40 polyA signal | no transcription terminator |
| px6062 | full length hCMV with Intron A | bGH polyA signal | human gastrin transcription terminator (hGT) |
| px6063 | hEf1α promoter with Intron A | bGH polyA signal | human gastrin transcription terminator (hGT) |

The best 18 stable clones obtained for each vector were identified and productivity of the clones was tested in batch analysis.

The exchange of the SV40 polyA signal by the combination of the bGH polyA signal and the hGT enhances average productivity: in case of the hEF1α promoter by about 19 % (505 µg/ml for vector px6063 versus 426 µg/ml for vector 6052) and in the case of the full length hCMV promoter by about 71 % (333 µg/ml for vector px6062 versus 195 µg/ml for vector px6051).

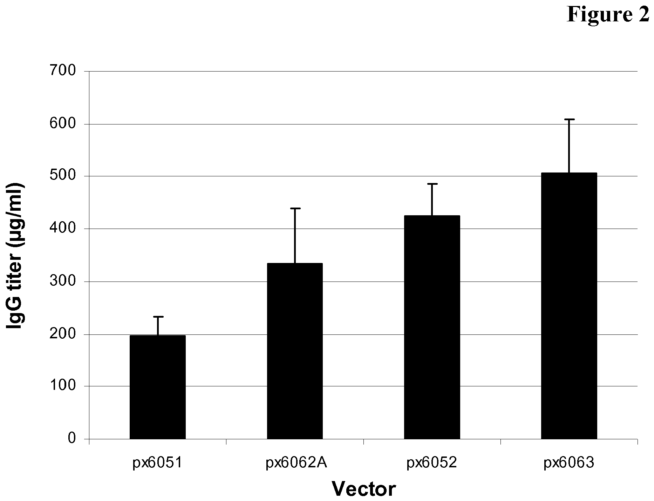

The increase in productivity can also be seen on top clone level (+ 36 % for vector px6063 (693 µg/ml) versus vector px6052 (511 µg/ml) and + 102 % for vector px6062 (529 µg/ml) versus vector px6051 (262 µg/ml), values are calculated for the three best clones each vector) (see Figure 2).

Thus, it has been found that the combination of the hEF1alpha promoter with bGH polyA signal and the hGT enhances productivity in stable transfection using a bidirectional vector organization with the expression cassette for the selection marker in one transcription direction and the expression cassettes for the light chain upstream of the expression cassette for the heavy chain in the respective other transcription direction (see px6052).

Further, it has been found that the combination of the bGH polyA signal and the hGT enhances productivity in stable transfection for the hCMV promoter using either an unidirectional vector organization with the expression cassette for the light chain upstream of the expression cassette for the heavy chain which in turn is located upstream of the expression cassette of the selection marker (5'->3' orientation of LC-HC-SM expression cassettes) (see px9005), or a bidirectional vector organization with the expression cassette for the selection marker in one transcription direction and the expression cassettes for the light chain upstream of the expression cassette for the heavy chain in the respective other transcription direction (see px6007).

The combination of the bGH polyA signal and the hGT was also employed in a transient transfections approach. Vectors px6062 and px6063 (containing the full length hCMV promoter or the hEF1α promoter with the combination of the bGH polyA signal and the hGT) were directly compared to vectors px6051 and px6052 (containing the containing the full length hCMV promoter or the hEF1α promoter with the SV40 polyA signal).

In contrast to stable transfections the combination of the bGH polyA signal and the human gastrin terminator (hGT) does not increase productivity in transient transfections, neither in the case of the full length human CMV promoter with Intron A nor in the case of the human EF1α promoter with Intron A (2.38 µg/ml for vector px6052 versus 2.32 µg/ml for vector px6063). In the case of the full length human CMV promoter with Intron A the combination of the bGH polyA signal and the human gastrin terminator (hGT) resulted in a productivity of 2.74 µg/ml for vector px6062A and 3.64 µg/ml for vector px6051.

In cells obtained by transient transfection it has been found that the combination of short hCMV promoter with bovine growth hormone polyA signal sequence and human gastrin terminator results in an improved antibody expression yield.

In cell lines obtained by transfection and selection of stable cell clones it has been found that the combination of bovine growth hormone polyA signal sequence and human gastrin terminator independently of the used promoter results in an improved antibody expression yield.

In vector p5068 the expression of the genes encoding for both the light (LC) and the heavy (HC) chain of the antibody is driven by the short human CMV promoter (hCMV). This promoter is active in a broad range of cell types and is commonly used in mammalian expression plasmids. As promoter activity is strongly cell type dependent and as it is known that the hCMV promoter is sensitive to gene silencing effects as promoter methylation possibly stronger and/or more resistant promoters in CHO-K1 cells exist.

Expression vectors comprising different promoters were constructed: i) comprising the short-length hCMV without Intron A, ii) the full-length hCMV promoter with Intron A, iii) the full-length ratCMV promoter with Intron A, and iv) the hEF1 alpha promoter with Intron A.

Vectors containing the full length hCMV promoter (px6051), the hEf1α promoter (px6052) and the rat CMV promoter (px6053) were constructed and used for transient and stable transfections.

| **vector** | **promoter** | **polyA signal** | **transcription terminator** |
|---|---|---|---|
| p5068 | short human CMV without Intron A | SV40 polyA signal | no transcription terminator |
| px6051 | full length human CMV with Intron A | SV40 polyA signal | no transcription terminator |
| px6052 | hEf1a promoter with Intron A | SV40 polyA signal | no transcription terminator |
| px6053 | rat CMV promoter with Intron A | SV40 polyA signal | no transcription terminator |

The expression vectors p5068, px6051, px6052, and px6053 were transiently transfected into CHO-K1 cells by nucleofection and four days after transfection cell culture supernatant was harvested and productivity was determined by ELISA.

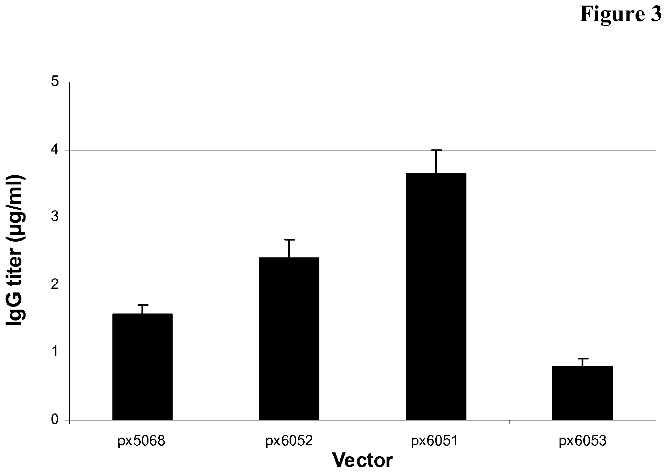

Vectors containing the hEF1a promoter with Intron A (px6052) or the full length hCMV promoter with Intron A (px6051) had an increased productivity by 53 % and 134 %, respectively, compared to expression vector p5068 (3.64 µg/ml for vector px6051, 2.38 µg/ml for vector px6052, 1.56 µg/ml for vector p5068). Vector px6053 containing the ratCMV promoter however showed an approximately 50 % reduced productivity (0.8 µg/ml for p5068) (see Figure 3).

Vectors containing the full length human CMV promoter (vectors px6051 and px6062) or the hEF1 alpha promoter with Intron A (vectors px6052 and px6063) were also used for stable transfections.

The expression vectors p5068, px6051, px6052, and px6053 were transfected into CHO-K1 cells by nucleofection and stable pools were selected. Productivity of the pools was analyzed in batch analysis.

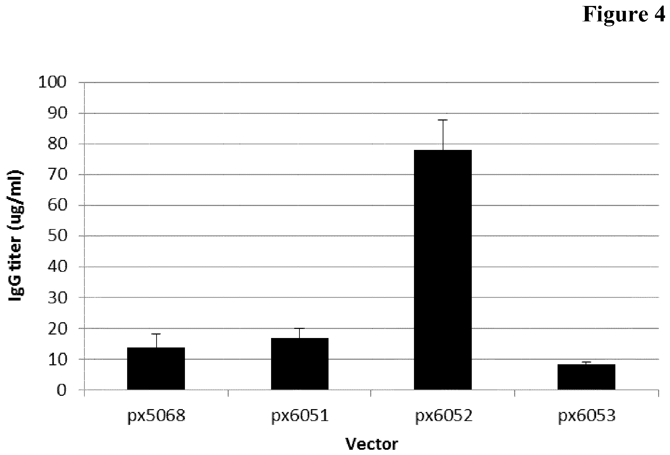

Batch analysis of stable pools showed that antibody titers from cells transfected with the vector containing the hEF1 alpha promoter with Intron A (vector px6052) were more than 4 fold higher than those of cells transfected with the vector containing the short-length hCMV promoter (vector p5068) or with the vector containing the full-length hCMV promoter with Intron A (vector px6051) (78 µg/ml for vector px6052, 14 µg/ml for vector p5068, 17 µg/ml for vector px6051, 8.46 µg/ml for vector px6053) (Figure 4).

Using the expression vector p5068, px6051, and px6052 stable clones were generated. Additionally vectors px6062 and px6063 (both containing the combination of the bGH polyA signal and the human gastrin terminator instead the SV40 polyA signal) were used.

| **vector** | **promoter** | **polyA signal** | **transcription terminator** |
|---|---|---|---|
| p5068 | short hCMV promoter with Intron A | SV40 polyA signal | no transcription terminator |
| p6051 | full length hCMV with Intron A | SV40 polyA signal | no transcription terminator |
| px6052 | hEf1a promoter with Intron A | SV40 polyA signal | no transcription terminator |
| p6062 | full length hCMV with Intron A | bGH polyA signal | human gastrin transcription terminator (hGT) |
| px6063 | hEf1a promoter with Intron A | bGH polyA signal | human gastrin transcription terminator (hGT) |

Batch analysis of the best 54 clones obtained for each vector showed that the average productivity from cells transfected with the vector px6052 was 316 µg/ml, and for vector p5068 341 µg/ml. Clones generated with the vectors px6051 and px6062 containing the full length hCMV promoter had an average productivity of 141 µg/ml for vector px6051 and 220 µg/ml for vector px6062.

The average productivity of the 54 tested clones transfected with the vector px6063 (containing the hEF1 alpha promoter in combination with the bGH polyA signal and the hGT) in batch analysis had a productivity of 367 µg/ml. The best clones with the highest titer are derived from the vector px6063 (748 and 731 µg/ml, respectively).

It has been found that clones generated with the vector containing the human EF1α promoter (px6052 and px6063) show a reduced number of low producing clones.

The clones were tested for stability of antibody production. Twelve clones obtained with the vectors p5068 and px6052 were cultivated in the presence and in the absence of Hygromycin B for three passages.

In the absence of selection pressure (Hygromycin B) for three passages, a 35 % decrease in the average productivity of the 12 tested clones was observed for the short-length hCMV promoter lacking intron A (p5068; 264 µg/ml versus 410 µg/ml). In contrast only an 18 % decrease in the average productivity was observed for the hEF1-alpha promoter with intron A (px6052; 256 µg/ml versus 312 µg/ml).

In the presence of selection pressure, the clones obtained with vector p5068 showed a decrease in average productivity of 27 % (298 µg/ml versus 410 µg/ml) and titers from clones obtained with vector px6052 containing the hEF1-alpha promoter with intron A decreased by 15 % (266 µg/ml versus 312 µg/ml).

The number of stable clones, transfected with the vector p5068 containing the short-length hCMV promoter lacking intron A or with the vector px6052 containing the hEF1-alpha promoter with intron A, after three passages with or without selection pressure was determined. To determine whether a clone is "stable" after three passages, IgG titers from passage zero were set to 100 % and a threshold of 80 % was defined. Clones showing relative IgG titers after 3 passages with or without selection pressure that are lower than 80 % of the IgG titer at passage zero (passage 0) were defined as instable.

Relative antibody titers after three passages, either with or without selection pressure, from all clones transfected with p5068 dropped below 80 % compared to the antibody titer of passage zero. A stable cell clone is a clonal cell population that produces an antibody titer that is 80 % or more compared to the antibody titer of passage zero in the presence of selection pressure as well as in absence of selection pressure.

Relative titers after three passages, either with or without selection pressure, from clones transfected with px6052 dropped below 80 % compared to the IgG titer of passage zero in 8 of 12 cases, but 4 clones were defined as "stable". In contrast to clones generated with the vector p5068 8 of 12 clones generated with the vector px6052 are "stable" in the presence of selection pressure. In contrast only one clone derived by the vector p5068 is stable in the presence of selection pressure.

Vectors px6014, px6014A and px6014B contain the hEF1a promoter in front of the light chain of the antibody and the short hCMV promoter in front of the heavy chain. These vectors vary in the 5' UTR of the light chain (px6014: 5' UTR of the hEF1α promoter containing the PmeI restriction site; px6014A: 5' UTR of the hEF1α promoter without PmeI restriction site; px6014B: 5' UTR of the hEF1α promoter plus the 5' UTR of the vector p5068). The vectors were transiently transfected in CHO-K1 cells using nucleofection.

Vectors px6014A and px6014B show a 20 % and 40 %, respectively, enhanced productivity compared to the vector px6014 (2.01 µg/ml for vector px6014B, 1.71 µg/ml for vector px6014A, 1.41 µg/ml for vector px6014).

It has been found that in transient transfections vectors containing the human EF1α promoter or the full length human CMV promoter (both with Intron A) show an improved productivity compared to vector p5068.

It has been found that stable pools generated with vectors containing the human EF1α promoter show an improved productivity in batch analysis compared to vector p5068.

It has been found that cell clones obtained by stable transfection with vectors containing the human EF1α promoter with Intron A show a reduced number of low producing clones.

It has been found that cell clones obtained by stable transfection with vectors containing the full length human CMV promoter with Intron A show a strongly reduced productivity both in average and on top clone level.

Thus, it has been found that the combination of the bGH polyA signal and the hGT enhances productivity compared to SV40 polyA in stable transfection independently of the used promoter using a bidirectional vector organization with the expression cassette for the selection marker in one transcription direction and the expression cassettes for the light chain upstream of the expression cassette for the heavy chain in the respective other transcription direction (see px6052).

Further, it has been found that the combination of the bGH polyA signal and the hGT enhances productivity in stable transfection for the hCMV promoter using an unidirectional vector organization with the expression cassette for the light chain upstream of the expression cassette for the heavy chain which in turn is located upstream of the expression cassette of the selection marker (5'-> 3' orientation of LC-HC-SM expression cassettes).

### IMPROVEMENT OF THE SELECTION PROCESS

Due to the high number of none and low producing clones and the low stability of gene expression cell line development is currently very time-consuming and cost intensive. In thousands of clones you normally find only a few stable "high producers".

Without being bound by theory a possible reason for the low selectivity and stringency of a selection strategy can be the separate expression of antibody and selection marker in the used vector system that does not exert selection pressure on the antibody expression (see e.g. vector p5069).

It has been found that by the use of an IRES element this problem can be overcome.

IRES elements are DNA elements that function - on mRNA level - as an internal ribosome entry site, and therefore allow the expression of two genes from one mRNA.

The IRES-linked expression of the selection marker and the antibody chain exerts selection pressure on total antibody expression (i.e. one mRNA coding for both the selection marker and the antibody chain) and, thus, warrants the selectivity of selection. The stability of gene expression can be increased.

The use of an IRES element with weak IRES activity allows a weak expression of the selection marker. This weak expression of the selection marker can increase the stringency of selection.

By the IRES-linked co-expression of selection marker and antibody chain the selection process can be improved by
- increasing the number of producing cells,
- enhancing the stability of gene expression, and
- enhancing the stringency of the selection process.

For the IRES-linked co-expression of antibody and selection marker the used IRES element has to fulfill two requirements:
- the IRES element may not or only minimal influence mRNA stability / antibody expression, and
- the IRES element has to show weak IRES activity so that the selection marker is only slightly expressed.

The light and the heavy chain encoding nucleic acid were linked by several IRES elements, the EV71-IRES, the ELF4G-IRES and the EMCV-IRES element (vectors px6015A, px6015B, and px6015C). The expression cassette comprises in 5' to 3' direction the human CMV promoter, the light chain encoding nucleic acid, the IRES, the heavy chain encoding nucleic acid, and the polyA site.

The vector p5068 without IRES element as reference and the vectors px6015A, px6015B, and px6015C, were transiently transfected in CHO-K1 cells and productivity was determined by ELISA.

Vectors px6015B and px6015C (containing the ELF4G and the EMCV-IRES respectively) show an IgG expression of 0.1 µg/ml to 0.15 µg/ml. Vector p5068 shows an IgG expression of 2 µg/ml. Vector px6015A containing the EV71-IRES shows a productivity of 1.7 µg/ml.

Alternatively the selection marker Neomycin can be linked by the IRES element directly to the heavy chain of the antibody. The vector comprises in 5' to 3' direction the elements human CMV promoter, light chain encoding nucleic acid, polyA site, human CMV promoter, heavy chain encoding nucleic acid, IRES element, neomycin selection marker nucleic acid, and polyA site.

The vectors px6010A, px6010B and px6010C were transiently transfected in CHO-K1 cells as described and productivity was determined by ELISA.

Vector px6010C containing the EMCV-IRES shows a productivity of 13 µg/ml. The vector p5069 shows a productivity of 14 µg/ml. Vectors containing the EV71-IRES (vector px6010A) or the ELF4G-IRES (vector px6010B) show a productivity of 4.3 µg/ml and 2.4 µg/ml,respectively.

Thus, it has been found that the EMCV-IRES element fulfills the requirements that are necessary for the IRES-linked expression of a selection marker:
- a weak IRES activity (so that the selection marker is only slightly expressed)
- at most a minimal influence of the IRES element - respectively the IRES-linked expression of the selection marker - on antibody expression.

Thus, the selection marker Neomycin (mediates resistance to G418) can be linked to the heavy chain encoding nucleic acid by the EMCV-IRES.

Vectors p5069 and px6010C were tested for productivity and stability in stable transfections, both on pool and on single clone level.

The vectors were transfected into CHO-K1 cells by nucleofection technology. Stable pools were selected and productivity of the pools was analyzed in batch analysis.

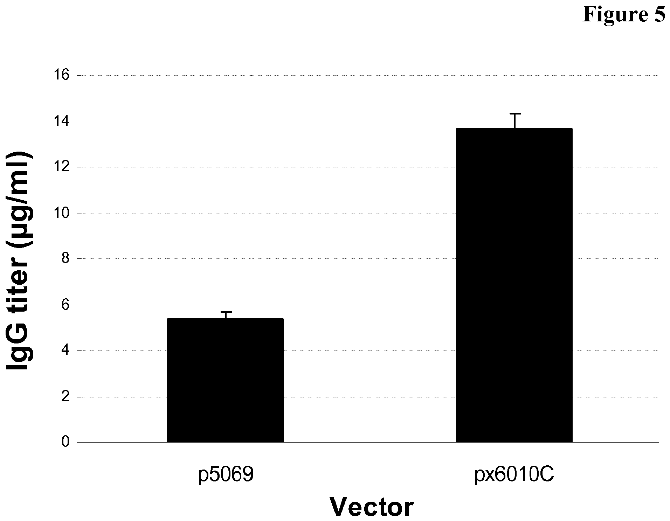

Pools generated with the vector px6010C show a productivity in batch analysis of 14 µg/ml. Pools generated with the vector p5069 show a productivity in batch analysis of 5.4 µg/ml (Figure 5).

It has been found that pools generated with the vector px6010C consist of more producing cells (or more good producing cells) and/or stability of IgG expression is enhanced (compared to the vector p5069).

It has been found that the IRES-linked expression of the selection marker Neomycin in the vector px6010C also leads to an enhanced stability of the pool. Stable pools were cultured in the presence and in the absence of selection pressure for 30 generations. At the beginning (= generation 0) and at the end (= generation 30) of the stability test productivity of the pools was determined in batch analysis.

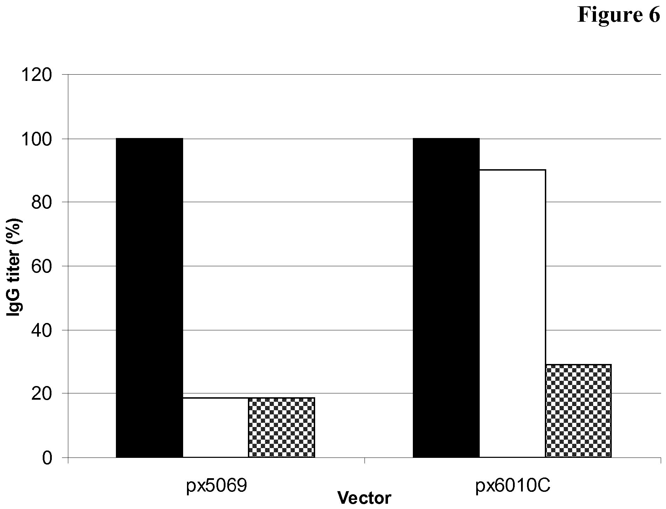

Pools generated with the vector px6010C show an enhanced stability of IgG expression in batch analysis compared to pools generated with the vector p5069. The productivity of pools obtained with the vector p5069 in batch analysis strongly decreased (> 80 %; values under the detection limit) after 30 generations both in the presence and in the absence of selection pressure. Productivity of pools generated with the vector px6010C also decreased about 70 % in the absence of selection pressure. In the presence of selection pressure however productivity of pools generated with the vector px6010C decreased only by 10 % (Figure 6).

Vector p5069 and vector px6010C were transfected into CHO-K1 cells by nucleofection and stable clones were generated as described above. Clones were screened and productivity of the best 15 clones each transfection was analyzed in batch analysis. Two independent transfections each vector were performed.

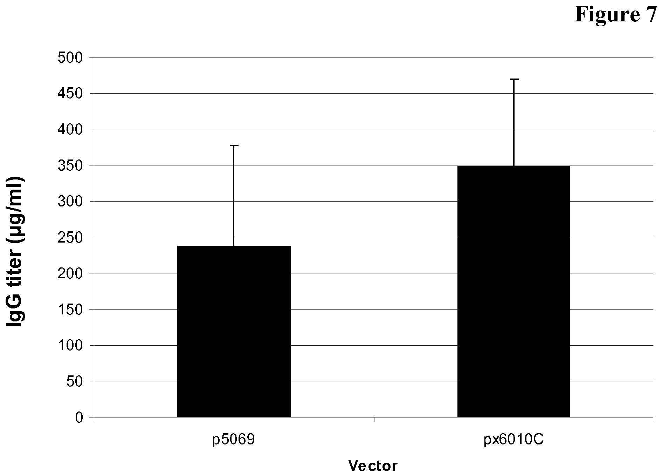

Clones generated with the vector px6010C show an average productivity of 348 µg/ml. Clones generated with the vector p5069 shows an average productivity of 239 µg/ml. The increased average productivity of clones generated with the vector px6010C is not due to better top clones but to a clearly decreased number of low producing clones. In contrast to the vector p5069 clones generated with the vector px6010C do not show lower productivities than 200 µg/ml in batch analysis (Figure 7).

The stability of antibody expression was tested for 17 clones (p5069) and 14 clones (px6010C), respectively, by cultivation in the presence and in the absence of selection pressure for 45 generations. Productivity of clones was measured at the beginning of the stability test (generation 0) and at generation 45 in batch analysis. IgG titer of generation 45 were compared to productivity of the clones at the beginning of the stability test.

The average productivity of the 17 clones generated with the vector p5069 decreased after 45 generations both in the presence and in the absence of selection pressure (approximately 43 % loss in productivity). The average productivity of the 14 clones generated with the vector px6010C decreased in the absence of selection pressure for approximately 45 %. However, in the presence of selection marker the decrease in productivity for these clones is 4 %.

In the presence of selection pressure only 6 of the 17 tested clones generated by the vector p5069 show a productivity that is still above 80 % of the productivity at generation 0. In contrast 10 of the 14 tested clones generated with the vector px6010C show a productivity that is above 80 % of the productivity at generation 0.

In the absence of selection pressure there is no significant difference in stability between clones generated with the vector p5069 and the vector px6010C respectively.

### IMPROVEMENT OF THE SELECTION AND SCREENING PROCESS

Due to intensive screening efforts cell line development is currently very time- and cost-intensive. In thousands of clones you generally find only a few stable high producers.

Currently several screening strategies for the identification of high producing clones exist:
- ELISA based screening strategies for the direct detection of the produced protein (time and cost intensive);
- fluorescence based screening strategies for the direct detection of the produced protein (time- and cost-intensive);
- FACS based sorting of cells co-expressing a quantitative screening marker (surface proteins or fluorescence marker as GFP) for the indirect detection/quantification of the produced protein.

In most of the cases the expression of the fluorescence marker is not linked to the expression of the antibody. This limits the dependence of fluorescence intensity and productivity, so there is often no good correlation between fluorescence intensity and productivity.

The GFP-protein (as example of a fluorescence marker) is stable and tends to accumulate. There is (in most cases) no good correlation between the expression level of the fluorescence marker in the cells (fluorescence intensity) and their productivity.

It has been found that by linking the antibody expression and the fluorescent marker expression a combined selection and screening strategy to identify cell clones having an enhanced stability and productivity but at the same time being simple and, thus, allowing a fast and easy identification of high producers.

It has been found that the IRES-linked expression of a fusion protein functions both as selection and as a quantitative screening marker.

A fusion protein of the green fluorescent protein (GFP) and the selection marker neomycin that is directly linked to the heavy chain of the antibody by an IRES element has been constructed. The green fluorescent protein and the selection marker neomycin in the fusion protein are separated by the PEST sequence (sequence corresponding to codons 423 - 449 of the mouse ornithine decarboxylase gene (mODC)). This PEST sequence serves on the protein level as a strong proteolytic signal sequence, thus clearly reducing the half-life of the protein.

The GFP-PEST-Neo fusion protein functions not only as selection but additionally as quantitative screening marker.

The use of the PEST sequence provides for an improved selection of clones due to the PEST mediated decreased half-life of the fusion protein (enhanced stringency of selection).

The use of the PEST sequence provides for a good correlation between the GFP fluorescence intensity of the fusion protein and the antibody expression level due to the IRES-linked co-expression of antibody and selection marker.

The use of the PEST sequence reduces accumulation of the fusion protein due to decreased half-life of the protein.

The GFP expression level of stable single clones was analyzed by FACS and correlated to the productivity in batch analysis. Populations with different GFP expressing levels from stable pools were sorted by FACS and the productivity of the sorted populations was analyzed.

IRES elements that fulfill the requirements for this approach:
- should provide weak IRES activity so that the fusion protein is only slightly expressed;
- shall not influence the antibody expression level.

IRES activity or strength was determined by linking the light and heavy chain expression by several IRES elements, the EV71-IRES, the ELF4G-IRES and the EMCV-IRES element (see vectors px6015A, px6015B, and px6015C). Vector p5068 (without IRES element) as reference and the vectors px6015A, px6015B, and px6015C, were transiently transfected in CHO-K1 cells and productivity was measured by ELISA.

Vectors px6015B and px6015C (containing the ELF4G and the EMCV-IRES, respectively) show an IgG expression of 0.1 to 0.15 µg/ml. An IgG expression of 2 µg/ml was shown for vector p5068. Vector px6015A containing the EV71-IRES showed a productivity of 1.7 µg/ml indicating that the EV71-IRES element has a strong IRES activity in CHO-K1 cells.

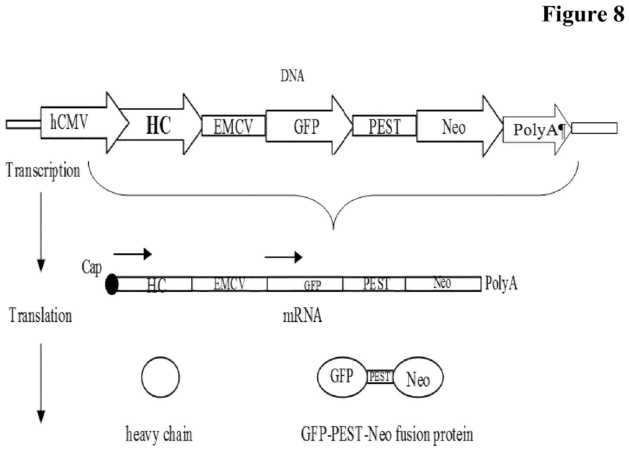

The GFP-PEST-Neo fusion protein is linked by the IRES element directly to the heavy chain encoding nucleic acid of the antibody (see Figure 8).

A construct in which the IRES element linked the selection marker to the heavy chain of the antibody comprised in vectors px6011A, px6011B and px6011C was transiently transfected in CHO-K1 cells as described and productivity was determined by ELISA.

Vector px6011C containing the EMCV-IRES shows an IgG productivity of 8.7 µg/ml and vector p5059 showed an IgG productivity of 10.8 µg/ml. Vector px6011A containing the EV71-IRES showed an IgG productivity of 3.9 µg/ml. Vector px6011B containing the ELF4G-IRES did not show relevant productivity.

Vector px6011C with the IRES-linked GFP-PEST-Neo fusion protein, vector p5069, and vector px6010C were tested in batch analysis of stable pools and stable clones.

Vectors p5069, px6010C, and px6011C were transfected into CHO-K1 cells by nucleofection. Stable pools were selected as described and productivity of the stable pools was analyzed in batch analysis.

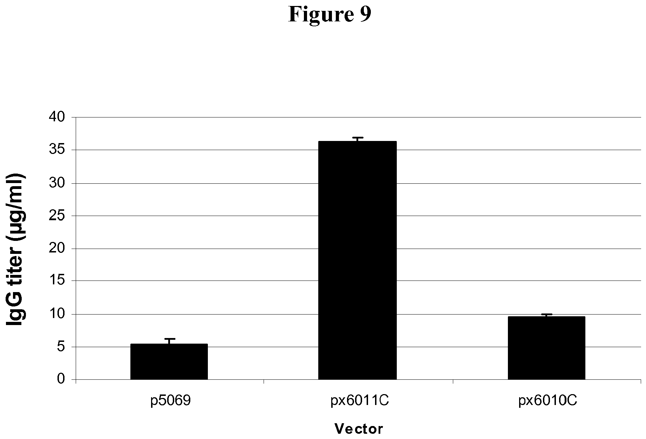

Stable pools generated with vector px6010C containing the IRES-linked selection marker showed an IgG productivity of 9.5 µg/ml. Stable pools generated with vector p5069 showed an IgG productivity of 5.4 µg/ml. Pools generated with the vector px6011C containing the IRES-linked fusion protein showed a productivity of 36.3 µg/ml (Figure 9).

These data suggest that pools generated with the vectors px6010C and px6011C apparently consist of much more producing or even good producing cells than the pools (generated with the vector p5069). An enhanced stability of IgG expression of/in the px6010C and px6011C pools can also contribute to the increased productivity.

To compare productivity of the expression vector p5068 with the vectors px6010C and px6011C in stable clones vectors were transfected into CHO-K1 cells by nucleofection. Stable clones were selected as described and clones were screened. Productivity of the best 15 clones each vector was analyzed in batch analysis.

For the vector p5068 95 of overall 3072 wells in 384 well plates showed an IgG production that is above 2 µg/ml (background). The IRES-linked expression of the selection marker (px6010C) doubled the number of producing clones/wells to 195, and in the case of the IRES-linked fusion protein (px6011C) the number of producing cells/wells was further increased to over 280.

Moreover, not only the number of producing clones but also the average productivity of the IRES containing clones (generated by the vectors px6010C and px6011C is higher (3.4 µg/ml for vector p5069, 4.2 µg/ml for vector px6010C, 8.1 µg/ml for vector px6011C for the best 100 clones each vector; 2.2 mg/ml for vector p5069, 3.0 µg/ml for vector px6010C, 5.1 µg/ml for vector px6011C for the best 250 clones each vector).

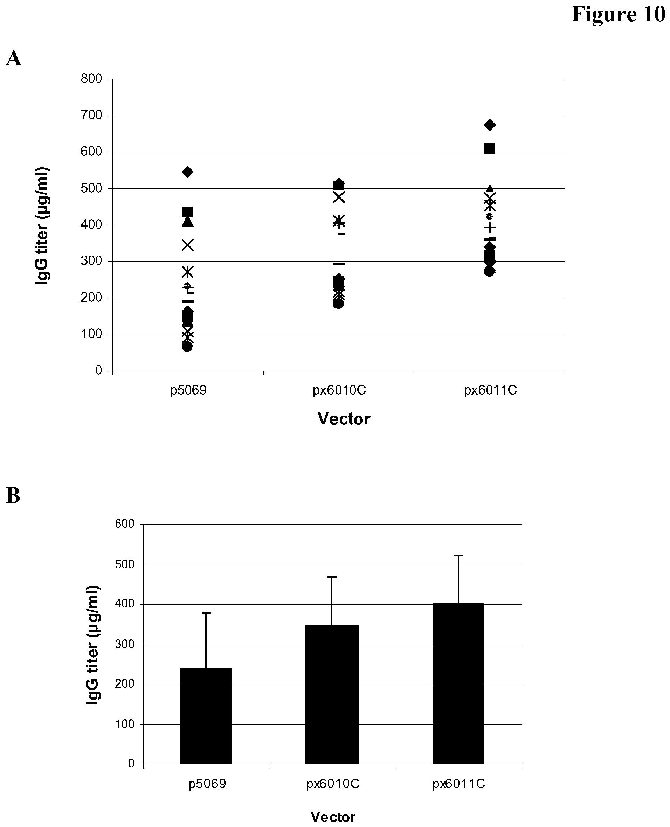

Clones generated with the IRES vectors px6010C and px6011C and p5059 show an average productivity in the 24 well screening of 212 µg/ml for vector px6011C, 178 µg/ml for vector px6010C, and 118 µg/ml for vector p5069 (determined in 24 well cultures with an undefined cell count at day 4 after cell split). For vectors px6010C and px6011C a reduced number of non or low producing clones and an increased number of good producing cells can be seen (Figure 10).

Batch analysis of single clones showed that the average productivity of the 15 best clones generated with the vector px6010C is 348 µg/ml and 239 µg/ml for vector p5069. The average productivity of the 15 best clones generated with the vector px6010C is 404 µg/ml. The clones with the highest overall titer derive from transfectants with the vector px6011C. Clones generated with the vector px6010C and the vector px6011C do not show lower productivities than 200 µg/ml in batch analysis.

In order to test the clones for stability of antibody expression, 14 to 19 clones obtained with each vector were cultivated in the presence and in the absence of selection pressure (G418) for 45 generations. Productivity of clones was measured at the beginning of the stability test (generation 0) and at generation 45 in batch analysis. IgG titer of generation 45 were compared to the productivity of the clones at the beginning of the stability test at generation 0 (values set to 100%).

The average productivity of the 17 clones generated with vector p5069 decreased after 45 generations both in the presence and in the absence of selection pressure (approximately 43 % loss in productivity). The average productivity of the 14 to 19 clones generated with the vectors px6010C and px6011C, respectively, also decreased in the absence of selection pressure (approximately 45 - 35 % loss in productivity), but in the presence of selection marker G418 the decrease in average productivity for these clones is only 0 to 4 % after 45 generations.

In the presence of selection pressure G418 6 of the 17 tested clones generated by the vector p5069 showed a productivity that is above 80 % of the productivity at generation 0. In contrast 10 of the 14 tested clones generated with the vector px6010C and 17 of the 19 tested clones generated with the vector px6011C showed a productivity that is above 80 % of the productivity at generation 0.

In the absence of selection pressure the clones generated with vectors p5069, px6010C, and px6011C show comparable behavior.

It has been found that IRES comprising vectors and, thus, the clones obtained therefrom have an enhanced stability in the presence of selection marker.

In the absence of selection marker however there is no difference in stability between clones obtained with an IRES containing vector or an IRES-free vector.

The GFP-PEST-Neo fusion protein is useful as a quantitative screening marker. The GFP fluorescent level of clones generated with the vector px6011C is predictive for their productivity.

The GFP expression level/fluorescence intensity of single clones were determined and results were correlated to the productivity of the clones in batch analysis, populations with different GFP expressing levels/fluorescence intensities were sorted from stable pools by FACS (1,000 cells each vector) and then productivity of these different populations was analyzed in batch analysis. Three different populations were sorted:
Population 1: no GFP expression Geometric mean (GM) of FL1-H (=GFP) 0-4
Population 2: low GFP expression level, GM 4-5.5
Population 3: high GFP expression GM 5.5 - 7.

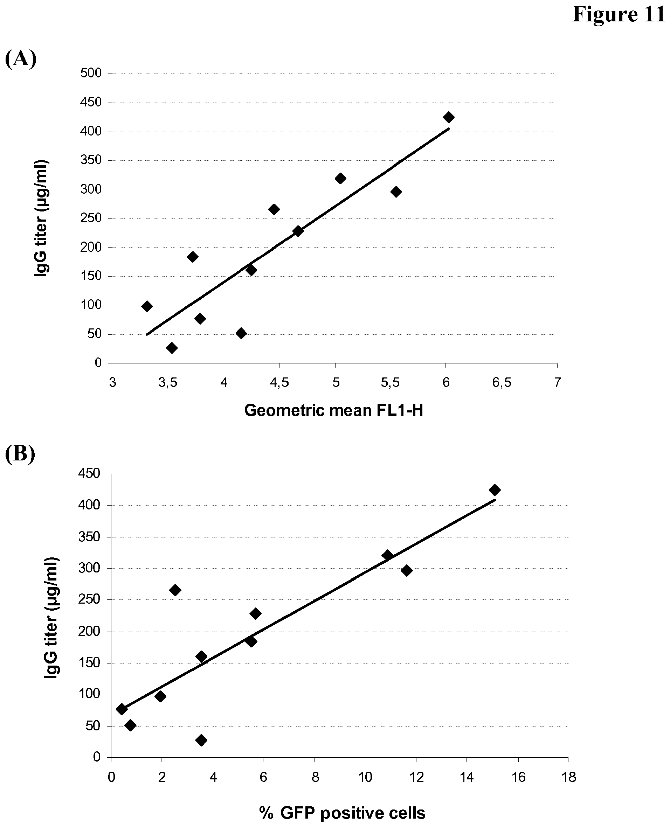

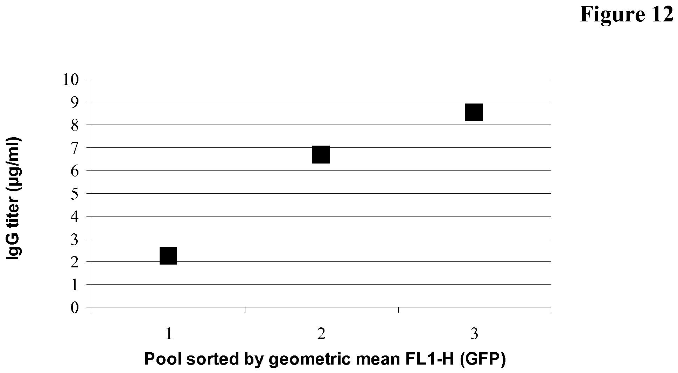

Batch and FACS analysis show that there is both on single clone and on pool level a good correlation between the GFP fluorescence of the clones/pools and their productivity. Clones or pools with a high GFP fluorescence generally show a higher productivity than cells showing a low GFP fluorescence. In general the productivity of a clone/pool increases with its fluorescence intensity (Figure 11 and 12).

High producer clones can be identified directly by a FACS based sorting of high GFP fluorescing single clones from stable pools. Single clones showing no or high GFP expression were sorted by FACS. Clones were expanded to shaken 6-well plates and productivity of clones was determined in batch analysis.

It has been found that the EMCV-IRES linked expression of the selection marker or the GFP-PEST-Neo fusion protein clearly enhances selectivity of selection (resulting in more producing clones) and it also enhances stringency of selection (average productivity of the producing clones is higher). Thus, the screening efforts are clearly reduced.

The stronger effect of the IRES-linked fusion protein (px6011C) in comparison to the IRES-linked selection marker (px6010C) is probably due to the PEST sequence in the fusion protein that mediates a reduced half time of the fusion protein and / or a less affinity of the fusion protein for the selection agent - both factors that apparently enhance stringency of selection.

### VECTOR ELEMENTS IN COMBINATION WITH VECTOR ORGANIZATION

The following vectors were tested in a CHO-K1 host cell line in transient transfections, in stable pools and some on single clone level.

| **vector** | **organization** | **promoter** | **polyA signal** | **transcription terminator** |
|---|---|---|---|---|
| px9001 | SM(3'-5')LC-HC | hCMV | SV40 polyA | not present |
| px9002 | LC-HC-SM | hCMV | SV40 polyA | not present |
| px9003 | LC-HC-SM | hEF1α | SV40 polyA | not present |
| px9004 | LC-HC-SM | hCMV | bGH polyA | not present |
| px9005 | LC-HC-SM | hCMV | bGH polyA | hGT |
| px9006 | LC-HC-SM | hEF1α | bGH polyA | not present |
| px9007 | LC-HC-SM | hEF1α | bGH polyA | hGT |
| px9010 | LC(3'-5')-HC-SM | hEF1α | bGH polyA | not present |
| px9011 | LC(3'-5')-HC-SM | hCMV | SV40 polyA | hGT |

Performances of different vectors in transient transfections were tested after nucleofection into CHO-K1 cells.

Vectors containing the human elongation factor 1 alpha promoter (hEF1α) (based on the vector organization LC-HC-SM) have an increased productivity of about + 34 % (px9003 versus px9002; SV40 polyA signal sequence) and + 30 % (px9006 versus px9004; bGH polyA signal sequence), dependent on the used polyA signal sequence, respectively, compared to the use of the hCMV promoter.

The addition of the human gastrin terminator (hGT) to the bGH polyA signal sequence has a positive effect on productivity for vectors containing the hCMV-(px9005 versus px9004; + 13 %).

Expression vectors based on the bidirectional expression of the light and the heavy chain of the antibody show improved performance. Product titers are about 2.7 to 3.4 fold increased compared to the control vector px9001 dependent on the used promoter (hEF1α or hCMV) and the used polyA signal sequence (SV40 or bGH polyA signal sequence).

It has been found that the use of human elongation factor 1 alpha promoter and the bGH polyA signal sequence has a positive effect on productivity in the vector organization LC-HC-SM (+ 50% ; compare px9006 and px9002) but not in vector organization LC(3'-5')-HC-SM (- 28 %; compare px9010 and px9011).

To compare the productivity of the expression vector px9002 with the vectors px9003 - 9007 vectors were transfected into CHO-K1 cells by nucleofection and stable pools were selected. Productivity of the pools was analyzed in batch analysis (see Figure 10).

Batch analysis of stable pools showed that antibody titers from pools transfected with the vector containing the human elongation factor 1 alpha promoter (px9003, px9006, px9007) were approximately 7-8 fold higher than those of cells transfected with the reference vector containing the short-length hCMV promoter (vector px9002) (compare 97.5 µg/ml, 112.5 µg/ml and 95.6 µg/ml and for vectors px9003, px9006 and px9007 versus 14.0 µg/ml for vector px9002.

Vectors px9001, px9002 and px9004 to px9007 were transfected into CHO-K1 cells by nucleofection and best single clones were identified by classical screening process. Productivity of best 36 clones each vector were analyzed in batch analysis and best 15 clones in batch analysis were tested in fed batch analysis.

The average productivity of the best 36 single clones generated with the vector px9001 in batch analysis is 356 µg/ml. Clones generated with the vector px9002 or with the vectors px9004 and 9005 (additionally containing the bGH PolyA signal (alone or in combination with the HGT) instead the SV40 PolyA signal) show a 37 % (px9002) respectively 61 % (px9004) to 53 % (px9005) increase in productivity compared to the control vector px9001. Clones of vector px9006 and px9007 show an increase in productivity of about 19 % and 7 % respectively.

In fed batch experiments best 15 clones obtained with each vector in batch analysis were tested in fed batch analysis over 14 days.

The average productivity of the best 15 single clones (generated with vector px9001) in fed batch analysis is 1345 µg/ml. Clones generated with the vector px9002 or with the vectors px9004 and px9005 (additionally containing the bGH polyA signal sequence (alone or in combination with the hGT) instead of the SV40 polyA signal sequence) show a 80 % (px9002) respectively 58 % (px9004) to 92 % (px9005) increase in productivity compared to the control vector px9001.

Beside an increased average productivity (see above) also the performance of the top clones is strongly improved. Vectors px9002, px9004 and px9005 show - with regard to productivity of the top 5 clones an increase of about 64 % (px9002), 50 % (px9004) and 88 % (px9005) compared to control vector px9001.

In the following the percentage of producing respectively non-producing cells for each of the different vectors was compared directly. 14.2 % of clones generated with vector px9001 produce antibody, in the rest of the resistant clones antibody expression is either silenced or clones have other defects.

The vector organization of vector px9002 almost doubled the percentage of producing cells (to 26.0 %). Vector additionally containing the bGH PolyA signal sequence alone instead the SV40 polyA signal sequence either alone (vector px9004) or in combination with the hGT (vector px9005) show an approximately 3 fold increase in percentage of producing cells (39 % and 43 %, respectively).

The use of the human elongation factor 1 alpha promoter instead of the hCMV promoter increased the number of producing cells up to 5 fold (more than 70 % of the clones obtained after the selection process do really produce antibody).

The 15 best clones obtained by transfection with vectors px9001-9007 (based on fed batch results) were cultivated in the presence and in the absence of Hygromycin B for 15 passages (= approximately 60 generations). Product titers of clones in batch analysis after 15 passages were compared with product titer of clones in batch at the beginning of stability test.

In the presence of selection pressure the change in product titer between 15 clones of each vector varies from - 14.7 % for vector px9007 and + 0.2 % for vector px9002 after 15 passages.

In the absence of selection pressure the decrease in product titer varies from 25.5 % for vector px9004 up to 5.9 % for vector px9005.

The number of clones that fulfill defined stability criteria such as > 80 % of product titer in batch analysis compared to values at starting point (G0) both in the presence and in the absence of selection marker varies from 4 - 10. Vectors px9005, px9007 and px9002 lead to the highest number of stable clones likewise in the presence and absence of selective pressure/selection marker (px9005: 10; px9007: 7; px9002:6)).

Thus, it has been found that the organization of vectors px9005 shows positive effects on stability and increase number of stable clones, especially in the absence of selection pressure.

It has been found that the combination of the bGH polyA and the hGT compared to the SV40 polyA without transcription terminator (hGT) clearly increases productivity of stable clones independent of the used promoter.

### Transient transfections:

- the use of the human elongation factor 1 alpha promoter (with Intron A) provides for an enhanced productivity (in LC-HC-SM organization)
- the use of the bovine growth hormone polyA signal sequence provides for an enhanced productivity compared to use of the SV40 polyA signal sequence
- the addition of the HGT to the bGH PolyA signal sequence results in an increased productivity in vectors containing the hCMV promoter
- vector organization LC(3'-5')-HC-SM results in improved expression

### Stable pools

- pools generated with vectors containing the hEF1α promoter show an enhanced productivity in batch analysis
- clones generated with vectors containing the hEF1α promoter show a reduced number of low producing clones
- clones generated with vectors containing the hEF1α promoter show a higher stability of IgG expression

### Single clones

- vector organization with downstream position of selection marker (LC-HC-SM) has a positive effect on productivity of stable single clones
- clones generated with vectors containing the bGH polyA signal sequence and the hGT have higher productivities and stabilities

### SUMMARY

Several different transcriptional relevant genetic elements and combinations thereof have been compared to a reference genetic element combination. On the basis of comparative transient experiments the following results have been obtained for the bidirectional vector organization with the expression cassette for the selection marker in opposite direction to the expression cassettes of the light and heavy chain (light chain expression cassette upstream of heavy chain expression cassette) (see table below).

| **genetic element in vector px5068** | **inserted element** | **result in transient experiments** | **results in stable experiments** |
|---|---|---|---|
| no transcription terminator | | | |
| | hGT transcription terminator | enhanced expression (+ 31 %) | enhanced expression (+ 37 %, stable pools); +23 % single clones |
| SV40 polyA signal | replaced by element | | |
| | bGH polyA signal | enhanced expression (+ 46 %) | stable pools (+ 38 %); single clones - 8% for the 15 best clones) |
| | bGH polyA + hGT | enhanced expression (+ 58%) | enhanced expression stable pools (+ 63 %); single clones + 40 % for 15 best clones) |
| short hCMV promoter | | | |
| | full length hCMV + Intron A | enhanced expression (+ 134 %) | pool: enhanced expression (+ 23 %) single clones: reduced expression (- 62 % for the best 15 clones) |
| | rat hCMV + Intron A | reduced expression (- 50 %) | reduced expression (- 40 %) |
| | hEF1α + Intron A | increased expression (+ 53 %) | pool: enhanced expression (+ 460 %) clones: - 16 % for best 15 clones |

Different promoters have been combined with the bGH polyA signal and the hGT transcription terminator (see table below).

| **genetic elements in vector px5068** | **replaced by element** | **result in transient experiments** | **results in stable experiments** |
|---|---|---|---|
| short hCMV promoter SV40 polyA signal | | | |
| | full length hCMV promoter with Intron A bGH polyA signal hGT transcription terminator | Increased expression + 70 % | Clones: reduced expression (- 34 % for best 15 clones) |
| | hEf1α promoter with Intron A bGH polyA signal hGT transcription terminator | Increased expression + 20 % | Clones in batch: similar expression |

Several transcription related genetic elements and combinations thereof have been compared to a reference vector (px9001, vector organization SM (3'-5' orientation)-LC-HC (5'-3' orientation)). On the basis of comparative experiments the following results have been obtained for the unidirectional vector organization with the expression cassette for the light and heavy chain (light chain expression cassette upstream of the heavy chain expression cassette) and for the selection marker in same direction (see table below) compared to a reference vector (px9001, bidirectional vector organization SM (3'-5')-LC-HC (5'-3')).

| **Reference vector px9001 (bidirectional, SM (3'-5')-LC_HC (5'-3')) or px9002 (unidirectional, LC-HC-SM (5'-3'))** | **inserted element** | **result in transient experiments** | **results in stable experiments** |
|---|---|---|---|
| | no differing elements but different vector organization (px9002, LC-HC-SM) | reduced expression of px9002 compared to px9001 (- 10 %) | single clones + 37 % in batch and + 80 % in fed batch analysis for px9002 compared to px9001 |

| **genetic element in vector px9002 (LC-HC-SM)** | **inserted element** | **result in transient experiments** | **results in stable experiments** |
|---|---|---|---|
| SV40 polyA signal | replaced by element | | |
| | bGH polyA signal (px9004) | enhanced expression (+ 15 % compared to px9002) | stable pools + 115 %; single clones + 61 % and + 58 % in batch and fed batch analysis for the best 36 and 15 clones, respectively (compared to px9001) |
| | bGH polyA + hGT (px9005) | enhanced expression (+ 30 % compared to px9002) | stable pool + 125 %; single clones + 53 % and + 92 % in batch and fed batch analysis for the best 36 and 15 clones, respectively (compared to px9001) |

| **Reference vector px9001 (bidirectional, SM (3'-5')-LC_HC (5'-3')) or px9002 (unidirectional, LC-HC-SM (5'-3'))** | **inserted element** | **result in transient experiments** | **results in stable experiments** |
|---|---|---|---|
| short hCMV promoter + SV40 polyA | | | |
| | hEF1α + Intron A + SV40 polyA (px9003) | increased expression (+ 34 % compared to px9002) | stable pool: enhanced expression, + 596 %; clones: single clones + 53 % and + 92 % in batch and fed batch analysis for the best 36 and 15 clones, respectively (compared to px9001) |
| | hEF1α + Intron A + bGH polyA (px9006) | increased expression (+ 50 % compared to px9002) | stable pool: enhanced expression + 704 %; single clones + 19 % and - 7 % in batch and fed batch analysis for the best 36 and 15 clones, respectively (compared to px9001) |
| | hEF1α + Intron A + bGH poly A + hGT (px9007) | increased expression (+ 47 % compared to px9002) | pool: enhanced expression + 583 % clones: single clones + 7 % and - 14 % in batch and fed batch analysis for the best 36 and 15 clones, respectively (compared to px9001) |

### Used vectors:

| **vector** | **promoter** | **polyA signal** | **transcription terminator** |
|---|---|---|---|
| px5068 | short hCMV without Intron A | SV40 polyA signal | no transcription terminator |
| px6001 | short hCMV without Intron A | SV40 polyA signal | hGT transcription terminator |
| px6008 | short hCMV without Intron A | bGH polyA signal | no transcription terminator |
| px6007 | short hCMV without Intron A | bGH polyA signal | hGT transcription terminator |
| px6051 | full length hCMV with Intron A | SV40 polyA signal | no transcription terminator |
| px6052 | hEF1a promoter with Intron A | SV40 polyA signal | no transcription terminator |
| px6053 | rat CMV promoter with Intron A | SV40 polyA signal | no transcription terminator |
| px6062 | full length hCMV with Intron A | bGH polyA signal | hGT transcription terminator |
| px6063 | hEF1α promoter with Intron A | bGH polyA signal | hGT transcription terminator |

It has been found that an increased expression (productivity) using the vector elements/element combination as reported herein can be achieved:

| | |
|---|---|
| - human CMV without Intron A: | 100 % (reference) |
| - human CMV with Intron A: | transient 234 %, |
| | stable pool 123 %, |
| | stable clones 38 % |
| - rat CMV with Intron A: | transient 50 %, pool 60 % |
| - human EF1α with Intron A: | transient 153 %, |
| | pool 564 %, |
| | stable clones: |
| | 84 % (SV40 polyA) |
| | approx. 100 % (bGH and hGT) |
| - human EF1α with Intron A and: | + 40 % (to human EF1α) |
| optimized 5'UTR | |
| - MPSV: | 29 % |
| - bGH polyA: | transient 146 %, |
| | stable 92 % |
| - hGT: | transient 131 %, |
| | stable pools 138 %, single clones 123 % |
| - bGH polyA and hGT: | transient 158 %, |
| | stable pools 163 %, single clones 140 % |

human CMV promoter:
   Xu et al., J. Control. Release, 81 (2002) 155-163.
   Xia et al., Prot. Expr. Purif. 45 (2006) 115-124.
rat CMV promoter:
   Xia et al., Prot. Expr. Purif. 45 (2006) 115-124.
human EF1α promoter:
   Teschendorf et al., Anticancer Res. 22 (2002) 3325-3330.
   Li et al., J. Immunol. Methods 318 (2007) 113-124.
MPSV promoter:
   Xia et al., Prot. Expr. Purif. 45 (2006) 115-124.
   Artelt et al., Gene 68 (1988) 213-219.
   Stocking et al., Proc. Natl. Acad. Sci. USA 82 (1985) 5746-5750.
   Lin et al., Gene 147 (1994) 287-292.
MPSV-CMV hybrid promoter:
   Liu et al., Anal. Biochem. 246 (1996) 150-152.

A high selectivity and high stringency selection process can be provided by using an IRES-linked expression cassette for the expression of the selection marker:
- selection pressure on antibody expression results in high selectivity
- linked expression of antibody and selection marker results in high selectivity
- it has been found that use of an IRES element with weak activity results in high stringency, i.e. high antibody production and low selection marker production
- linking the expression of antibody and selection marker by IRES elements
- identification of IRES elements (EMCV/Gtx) with weak activity which do alter IgG expression marginally
- use of a fusion protein working as selection and as screening marker
- bifunctional GFP-Neomycin fusion protein
- PEST sequence of the ornithine decarboxylase is a strong proteolytic signal sequence and confers a reduced half-life of the protein
- IRES linked expression of the fusion protein results in high selectivity
- short half-live by proteolytic signal sequence results in high stringency
- due to weak expression and short half-life of the fusion protein a strong expression is required
- rapid identification of high producers by FACS (sorting of high GFP expressing clones provides for selection of high producers)

Selection marker linked to heavy antibody chain by different IRES elements

| | | |
|---|---|---|
| - | px5068 (without IRES): | 100 % antibody expression (reference) |
| - | Gtx-IRES: | 20-27 % |
| - | EMCV-IRES | 81-94 % |
| - | EV71-IRES | 20-36 % |
| - | ELF4G-IRES | 3-17 % |
| - | Gtx-IRES (synthetic) | 88 % |

GFP-Neo Fusion protein linked to heavy antibody chain by different IRES elements

| | Gtx | EV71 | ELF4G | EMCV |
|---|---|---|---|---|
| GFP expression: | + | +++ | - | + |
| antibody expression: | - | - | - | + |

Gtx-IRES:
   Komuro et al., EMBO J. 12 (1993) 1387-1401.
EMCV-IRES:
   Mountford et al., Proc. Natl. Acad. Sci. USA 91 (1991) 4303-4307.
EV71-IRES:
   Lee et al., Biotechnol. Bioeng. 90 (2005) 656-662.
ELF4G-IRES:
   Wong et al., Gene Ther. 9 (2002) 337-344.
Gtx (synthetic)-IRES:
   Chappell et al., Proc. Natl. Acad. Sci. USA97 (2000) 1536-1541.

It has been found that an increased expression (productivity) using the linking of the light chain expression cassette to the heavy chain expression cassette can be achieved by the EV71-IRES element:

| | |
|---|---|
| - px5068 without IRES: | 100 % antibody expression (reference) |
| - Gtx-IRES (synthetic): | 3 % |
| - EV71-IRES: | 82 % |
| - ELF4G-IRES: | 5 % |
| - EMCV-IRES: | 7 %. |

Disclosed herein is an optimized human elongation factor 1 alpha promoter comprising an optimized 5'UTR that has the sequence of SEQ ID NO: 06.

The following examples, figures and sequences are provided to aid the understanding of the present invention, the true scope of which is set forth in the appended claims.

### Sequences

| | |
|---|---|
| **SEQ ID NO**: 01 | short human CMV promoter without Intron A |
| **SEQ ID NO: 02** | short human CMV promoter without Intron A with 5'UTR |
| **SEQ ID NO: 03** | full length human CMV promoter with Intron A |
| **SEQ ID NO: 04** | full length human EF1 alpha promoter without Intron A |
| **SEQ ID NO: 05** | full length human EF1 alpha promoter with Intron A |
| **SEQ ID NO: 06** | short human EF1 alpha promoter with Intron A with 5'UTR |
| **SEQ ID NO: 07** | full length rat CMV promoter with Intron A |
| **SEQ ID NO: 08** | SV40 polyA signal sequence |
| **SEQ ID NO: 09** | bGH polyA signal sequence |
| **SEQ ID NO: 10** | hGT terminator sequence |
| **SEQ ID NO: 11** | SV40 promoter |
| **SEQ ID NO: 12** | PEST sequence of ornithine decarboxylase |
| **SEQ ID NO: 13** | nucleic acid sequence encoding GFP |
| **SEQ ID NO: 14** | neomycin selection marker |
| **SEQ ID NO: 15** | GFP-PEST-NEO fusion polypeptide encoding nucleic acid |
| **SEQ ID NO: 16** | EMCV-IRES |
| **SEQ ID NO: 17** | EV71-IRES |

### Figures

- **Figure 1**: Productivity of stable clones generated with the vectors p5068, px6001, px6008 and px6007. Shown is the average productivity of the best 15 clones obtained with each vector in batch analysis of a total of three independent transfections.
- **Figure 2**: Productivity of stable clones generated with the vectors px6051, px6062, px6052, and px6063. Shown is the average productivity of the best 18 clones each vector in batch analysis of a total of three independent transfections.
- **Figure 3**: Productivity of vectors p5068, px6051, px6052 and px6053 in transient transfections using the 96well shuttle system from Amaxa. Shown is the average productivity of eight independent transfections each vector on day 4 after transfection measured by ELISA.
- **Figure 4**: Productivity of different stable pools generated with the vectors p5068, px6051, px6052, and px6053 in batch analysis. Shown is the average productivity of three pools each vector on day 7.
- **Figure 5**: Productivity of stable pools generated with the vectors p5069 and px6010C. Shown is the average productivity of two (px5069) respectively three (px6010C) different pools each vector in batch analysis on day 7.
- **Figure 6**: Stability of gene expression in stable pools generated with the vectors p5069 and px6010C. Shown is the average productivity of two different pools each vector in batch analysis on day 7 at passage 0 (set to 100 %, black column) and at generation 30 with (white column) and without selection (patterned column) pressure (G418).
- **Figure 7**: Productivity of the best 15 clones generated by the vectors p5069 and px6010C. Average productivity of the best 15 clones each vector in batch analysis of a total of two independent transfections.
- **Figure 8**: Schematic overview on the vector design of vector px6011C mediating the IRES mediated expression of the GFP-PEST-NEO fusion protein. The GFP-PEST-Neo fusion protein is linked by the EMCV-IRES to the heavy chain of the antibody. The coding sequences of the heavy chain of the antibody and of the fusion protein are transcribed by the short human CMV promoter in one mRNA. The translation of this mRNA leads to the heavy chain of the antibody and to the GFP-PEST-NEO fusion protein.
- **Figure 9**: Productivity of different stable pools generated by the vectors p5069, px6011C and px6010C in batch analysis. Shown is the average productivity of two different pools each vector on day 7.
- **Figure 10**: Productivity of the best 15 clones generated by the vectors p5069, px6010C (vector expressing the selection marker Neomycin by the EMCV-IRES element that is linked to the heavy chain of the antibody) and px6011C (vector expressing the fusion protein of GFP-PEST-Neomycin by the EMCV-IRES element that is linked to the heavy chain of the antibody). (A) Productivity distribution of the best 15 clones each vector in batch analysis of a total of two independent transfections. (B) Average productivity of the best 15 clones each vector in batch analysis of a total of two independent transfections.
- **Figure 11**: Shown is the dependence of the GFP expressing level/fluorescence intensity and productivity in batch analysis for 11 clones generated with vector px6011C. Clones were picked randomly in the 24 well screening format, then expanded and finally analyzed in batch analysis. The geometric mean (GM) of the GFP fluorescence intensity and the percentage of GFP-positive cells of each clone were determined by FACS. (A) Dependence of GFP fluorescence intensity and productivity in batch analysis for 11 single clones. (B) Dependence of the percentage of GFP-positive cells each clone and productivity in batch analysis for 11 single clones.
- **Figure 12**: Productivity of stable pools with different GFP fluorescence intensities in batch analysis. Cells with different GFP expressing levels/fluorescence intensities (low (1), medium (2) and high (3)) were sorted by FACS. Pools were expanded and productivity of the pools was determined in batch analysis on day 7.

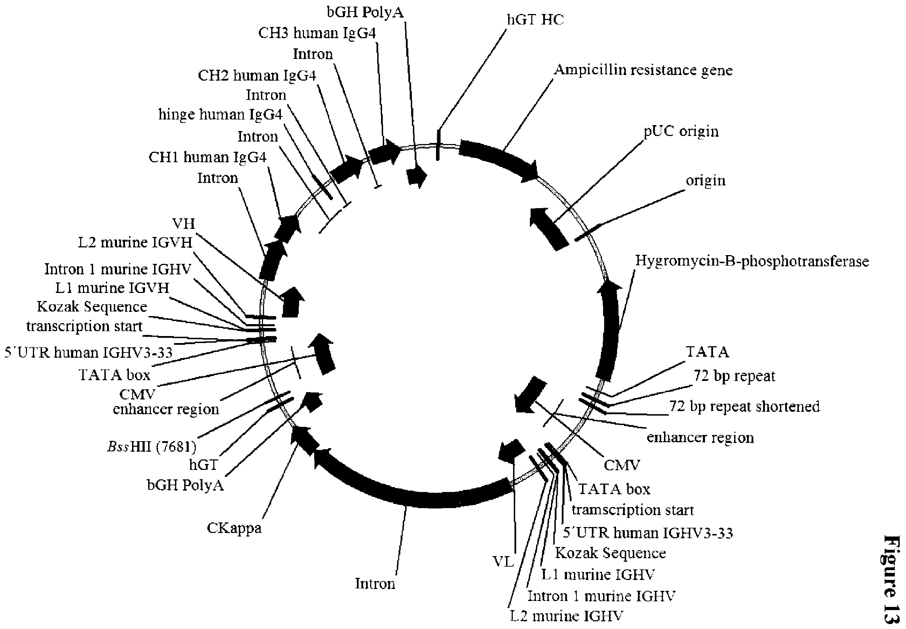

- **Figure 13**: Plasmid map of px6007.

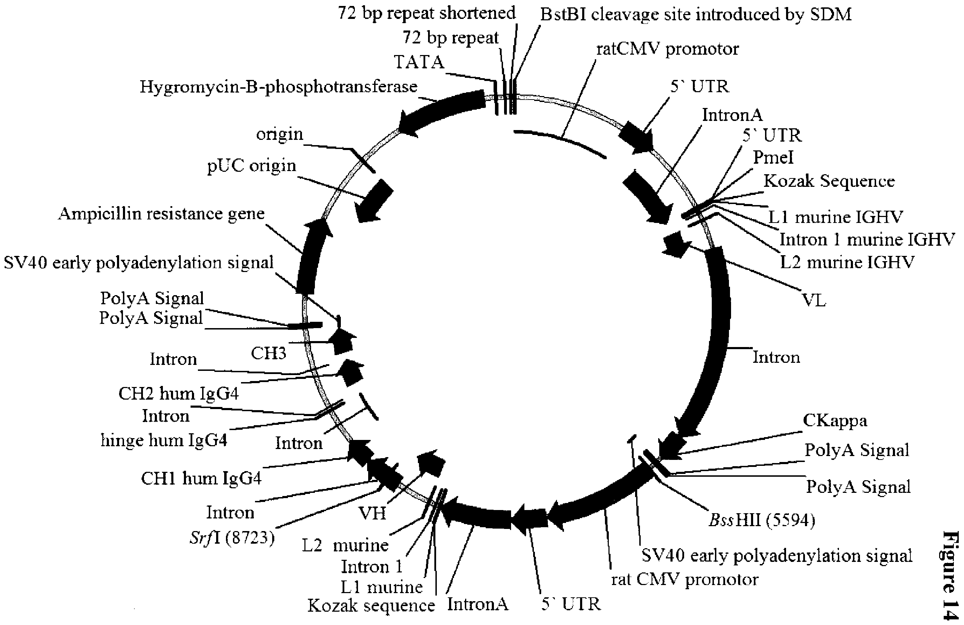

- **Figure 14**: Plasmid map of px6053.

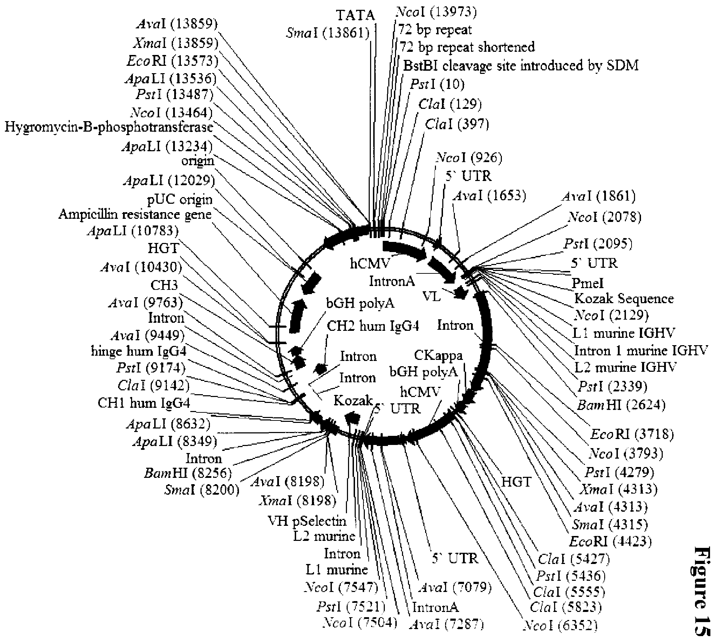

- **Figure 15**: Plasmid map of px6062.

### Examples

### Expression vector p5068 and p5069

Expression plasmids p5068 and p5069 comprise expression cassettes for the expression of an anti-P-selectin antibody (genomically organized expression cassette with retained exon-intron organization) as reported in WO 2005/100402.

The anti-P-selectin HuMab light and heavy chain encoding genes were separately assembled in mammalian cell expression vectors.

Thereby the gene segments encoding the anti-P-selectin HuMab light chain variable region (VL) and the human κ-light chain constant region (CL) were joined as were gene segments for the anti-P-selectin HuMab heavy chain variable region (VH) and the human γl-heavy chain constant region or the human y4-heavy chain constant region (CH1-Hinge-CH2-CH3).

General information regarding the nucleotide sequences of human light and heavy chains from which the codon usage can be deduced is given in: Kabat, E.A., et al., Sequences of Proteins of Immunological Interest, 5th ed., Public Health Service, National Institutes of Health, Bethesda, MD (1991), NIH Publication No 91-3242.

The transcription unit of the anti-P-selectin HuMab κ-light chain is composed of the following elements:
- the immediate early enhancer and promoter from the human cytomegalovirus (hCMV),
- a synthetic 5'-UT including a Kozak sequence,
- a murine immunoglobulin heavy chain signal sequence including the signal sequence intron,
- the cloned anti-P-selectin HuMab variable light chain cDNA arranged with a unique BsmI restriction site at the 5' end and a splice donor site and a unique NotI restriction site at the 3' end,
- the genomic human κ-gene constant region, including the intron 2 mouse Ig-κ enhancer (Picard, D., and Schaffner, W. Nature 307 (1984) 80-82), and
- the human immunoglobulin κ-polyadenylation ("poly A") signal sequence.

The transcription unit of the anti-P-selectin HuMab γl-heavy chain is composed of the following elements:
- the immediate early enhancer and promoter from the human cytomegalovirus (hCMV),
- a synthetic 5'-UT including a Kozak sequence,
- a modified murine immunoglobulin heavy chain signal sequence including the signal sequence intron,
- the cloned anti-P-selectin HuMab variable heavy chain cDNA arranged with a unique BsmI restriction site at the 5' and a splice donor site and a unique NotI restriction site at the 3' end,
- the genomic human γl -heavy gene constant region, including the mouse Ig µ-enhancer (Neuberger, M.S., EMBO J. 2 (1983) 1373-1378), and
- the human γl-immunoglobulin polyadenylation ("polyA") signal sequence.

Beside the anti-P-selectin HuMab κ-light chain or γl-heavy chain expression cassette these plasmids contain
- a hygromycin resistance gene,
- an origin of replication, oriP, of Epstein-Barr virus (EBV),
- an origin of replication from the vector pUC18 which allows replication of this plasmid in E. coli, and
- a β-lactamase gene which confers ampicillin resistance in E. coli.

### Recombinant DNA techniques

Cloning was performed using standard cloning techniques as described in Sambrook et al., 1999 (supra). All molecular biological reagents were commercially available (if not indicated otherwise) and were used according to the manufacturer's instructions.

### Nucleic acid synthesis

DNA of the different genetic elements was synthesized by Geneart AG, Regensburg.

### Nucleic acid sequence determination

DNA sequences were determined by double strand sequencing performed at SequiServe (SequiServe GmbH, Germany).

### DNA and protein sequence analysis and sequence data management

The Vector NTI Advance suite version 9.0 was used for sequence creation, mapping, analysis, annotation, and illustration.

### Cell culture techniques

CHO-K1 cells were grown in CD-CHO medium (Invitrogen Corp., Gibco^{®}, Cat. No. 10743-011) supplemented with 1x HT supplement (Invitrogen Corp., Gibco^{®}, Cat. No. 11067-030).

For the selection of stably transfected CHO-K1 pools/cells lines 400 to 800 µg/ml G418 or 200 to 400 µg/ml Hygromycin was added (Roche Diagnostics GmbH, Roche Applied Sciences, Germany, Cat. No.: 843555).

All cell lines were maintained in humidified incubators at 37 °C with 5 % CO₂ under constant agitation at 120 to 140 rpm/min. Every 3 to 4 days the cells were split into fresh medium. Density and viability of the cultures was determined using the Casey TT or Cedex Hires cell counter (Roche innovates AG, Bielefeld). Transfection of cells was performed by the Amaxa nucleofection technology (Lonza GmbH, Germany).

Furthermore standard cell culture techniques were applied as described e.g. in Bonifacino, J.S., et al., (eds.), Current Protocols in Cell Biology, John Wiley and Sons, Inc. (2000).

### Cell counting and determination of cell viability

### a) Electric field cell counting system (CASY)

The CASY^{®} Technology Cell Counter, Model TT (Roche Innovatis AG, Bielefeld) uses electric current for cell counting. The Pulse Area Analysis was used to get information from signals created when a cell passes through the measuring pore in a low voltage field. The structural integrity of the cell membrane is a degree for cell viability. Dyes such as trypan blue are therefore not needed for determination of viability.

### b) Automated trypan blue exclusion method (Cedex)

A Cedex HiRes system (Roche Innovatis AG, Bielefeld) was used to determine cell viabilities during pool selections and for automated cell counting.

Trypan blue is a dye that cannot enter cells through intact cell membranes. Only those cells are stained, and marked dead, which have a damaged cell membrane. The staining process, cell counting and graphical analysis of the results were performed automatically by the Cedex system by digital image recognition. Other measurement parameters are cell size, morphology and aggregation rate. With the multi sampler, up to 20 samples were measured consecutively.

### Plasmid preparation and quality check for accurate comparison of plasmids in transfections

Transfection efficacy and therefore productivity is strongly influenced by several factors such as DNA amount and quality. To ensure equal starting conditions for each vector the DNA amount and quality of all vectors were intensively checked before transfection.

### - Simultaneously preparation of expression vectors

All vectors were simultaneously prepared by the High Speed Maxi plasmid isolation Kit (Qiagen GMBH, Hilden) according to manufactures' instructions.

### - Phenol/chloroform purification and ethanol precipitation

All vectors were simultaneously purified by a phenol/chloroform purification. 500 µg each linearized plasmid DNA was mixed with 200 µl Tris-buffered 50 % (v/v) phenol, 48 % (v/v) chloroform, 2 % (v/v) isoamyl alcohol solution and centrifuged for 1 min. at 13,000 rpm. The upper aqueous phase was then transferred into a new tube and mixed with 200 µl 96 % (v/v) chloroform, 4 % (v/v) isoamyl alcohol and centrifuged for 1 min. at 13;000 rpm. The upper phase was again transferred into a new tube and mixed with 1/10 (total volume) 3 M sodium acetate (pH 5.2) and 2.5 times (total volume) 100 % ethanol. After mixing and incubating the reaction for 5 min. at room temperature, the mixture was centrifuged for 5 min. at 13,000 rpm in order to pellet the DNA. The supernatant was discarded and the pellet was washed with 900 µl 70 % (v/v) ethanol and incubated for 5 min. at room temperature. After a final centrifugation step at maximum speed for 5 min., the supernatants were discarded and the pellets were dried and resuspended with sterile H₂O.

### - DNA determination

The DNA amount of each vector was determined using the BioPhotometer (Eppendorf; Hamburg). DNA measurement was always performed in triplicates by using a 1:20 dilution in Tris pH 8.0).

### - Agarose gel

DNA quality of each plasmid was checked on a 0.8 % agarose gel. DNA degradation, vector conformations and DNA concentrations were determined. Vectors showing comparable quantities and qualities (no DNA degradation, similar supercoiled (ccc) forms, similar DNA amounts on gel) were used for transient and stable transfections.

### Transient transfections

All vectors were transfected in CHO-K1 cells by the Amaxa 96 well shuttle system (Lonza GmbH, Germany) according to manufactures' instructions. Each vector was transfected in 8 replicates. DNA amounts of transfected vector were normalized to equal molar amounts/copy numbers according to 1 µg of the reference expression plasmid (p5068 or p5069). To determine productivity cell free cell culture supernatant was analyzed for IgG titer on day 4 to 7 after transfection by a one-step universal ELISA (Dianova).

### Amaxa 96 well shuttle system:

CHO-K1 cells growing in spinner flasks were pelleted by centrifugation at 850 rpm for 5 min. and resuspended in culture medium. Circular plasmids were plated out in 96-well nucleofection plates at equimolar concentrations according to 1 µg of the reference expression vector p5068 or p5069. Cells were then added into the plates at a concentration of 4 × 10⁵ cells per well. The transfection was carried out by the Amaxa program DN-137. Cells were incubated for 10 min. after transfection and then transferred into 96 well flat-bottom incubation plates containing 200 µl culture medium. Cells were then statically cultivated. On day 4 to 6 after transfection IgG levels were determined using the one-step universal ELISA.

### Stable transfections and generation of recombinant CHO cell lines

Stable transfections were performed by the nucleofection technology (Amaxa Biosystems, Lonza cologne AG) according to manufactures' instructions. Before transfection plasmids were linearized by the restriction enzyme SgrA I. Each plasmid was transfected in duplicates or triplicates. 5 × 10⁶ cells and 1.2 pmol linearized plasmid were used per single transfection. (Nucleofector Kit T, Amaxa program A33).

For transfection cells were resuspended in the Nucleofector solution T and aliquoted into 2 ml tubes. After the addition of the plasmid, the transfection was carried out by applying the pulse. Cells were then transferred into T25 tissue culture flasks containing pre-warmed 4 ml fresh medium and 4 ml conditioned medium. Selective pressure was applied 24 hours post-transfection by adding 250 µg/ml Hygromycin B.

### Generation of stable pools

Vectors were transfected into CHO-K1 cells by Amaxa nucleofection technology and stable pools were selected using Hygromycin B or G418 as selection agent. Each transfection was performed in triplicates. For generation of stable pools, all plasmids were uniformly linearized by restriction digestion with SgrA I. The Nucleofector Kit T by Amaxa was used for carrying out stable transfections and each plasmid was transfected in triplicates.

Stable pools were established as follows: 5 × 10⁶ cells and 1.2 pmol linearized plasmid were used for each transfection. Cells were resuspended in Solution T and aliquoted into 2 ml tubes. After the addition of the plasmid, the transfection was carried out by applying the pulse (Amaxa program A33). The transfected cell pools were statically cultivated in T25 tissue culture flasks containing pre-warmed 4 ml fresh medium and 4 ml conditioned medium.

After 24 h post-transfection selection pressure was applied: Cells were centrifuged for 5 min. at 800 rpm, resuspended in 3 ml culture medium containing 300 µg/ml Hygromycin B. Cells were transferred into flat-bottom 6-well plates 3 days post-transfection. Cells were then cultivated for two weeks till cell viabilities dropped to a minimum and rose again over 99 %. Cell numbers and viabilities were constantly determined with a Cedex HiRes system (Innovatis, Bielefeld). During cultivation, cell debris was removed by centrifugation and cells were always resuspended in 3 ml fresh medium.

### Generation of stable clones using the Caliper robotic system

Vectors were transfected into CHO-K1 cells as described above. 48 hours after transfection selection pressure was applied (Hygromycin B or G418) and cells were seeded onto 384 well flat-bottom plates at a concentration of 350 to 700 cells per well using an automated high-throughput clone isolation system (Sciclone ALH 3000 workstation, Caliper Life Sciences GmbH, Mainz).

After 10 to 14 days the 384 well plates were screened for IgG levels using an ELISA based ultrahigh-throughput screening (ELSIA uHTS). From a primary screening the best producing clones were chosen and transferred into flat-bottom 96 well plates. After 3 to 6 days cells were screened for IgG levels in a second round. The best producing clones again were chosen and manually transferred into flat-bottom 24 well plates. After a further ELISA based screening step the best clones were chosen and transferred into flat-bottom 6 well plates. IgG levels in the 6-well plates were determined by ProtA measurement to identify the final best clones for batch culture in shaken 6 well plates.

### Batch analysis of pools/single clones

In order to detect differences in productivity and stability, cell numbers of the clones/pools were counted using a Casey cell counter and uniformly seeded into flat-bottom 6 well plates at a concentration of 3 x 10⁵ cells/ml and a total volume of 3.0 ml. All batch cultures were cultivated for 12 days and cell culture supernatants were screened for human IgG levels at day 4, 7, 9, 11 or 12.

### IgG quantification

The IgG titer in transient experiments and in the screening formats (384 well to 24-well) were determined by using the one-step universal ELISA. Productivity of stable pools and stable single clones in batch experiments were determined by Protein A HPLC.

### One-step universal ELISA

A one-step universal ELISA (Dianova) was used to determine human IgG levels from cell culture supernatants. A standard curve was prepared using serial dilutions of an anti-P-Selectin antibody (F. Hoffmann-La Roche AG, Basle, Switzerland) with a range of 0.3125-20 ng/ml using dilution buffer (PBS + 5 % (w/v) RPLA1). 95 µl antibody-mix containing 0.5 µg/ml biotinylated F(ab')₂-anti-human Fc antibody (Jackson laboratories) and 0.1 µg/ml peroxidase conjugated F(ab')₂-antihuman Fcγ antibody (Jackson laboratories; Suffolk) was added to streptavidin-coated 96-well MTP (StreptaWell, Roche Diagnostics GmbH). 5 µl of 1:20.000 diluted cell culture supernatant was added to the plates and incubated for 1 hour. Antibody coated plates were washed three times with 200 µl washing buffer (PBS + 0.05 % (v/v) Tween²⁰). 100 µl ABTS (Roche Diagnostics GmbH, Mannheim, Germany) was added to the plates and the absorbance was measured at 405 nm with a reference wavelength of 492 nm.

### ProtA-measurement

The IgG titer of batch analysis were determined by Protein A using a HPLC based chromatography in combination with the one-step universal ELISA.

### FACS

Fluorescence-activated cell sorting was used to determine transfection efficiencies (based on GFP expressing cells) or GFP expression levels of stably or transiently transfected cells. In general 5 × 10⁶ cells each clone or pool were measured using a FACSCalibur Flow Cytometer (BD Biosciences, San Diego, CA). Forward and sideward scatter data were used to determine cell size, viability and cell morphology.

## Claims

1. A method for selecting a recombinant stable transfected mammalian cell comprising the following steps:
a) transfecting a mammalian cell with an expression vector comprising
- a first expression cassette comprising in 5' to 3' direction a hCMV promoter, a nucleic acid encoding an antibody light chain, a bGH polyA signal sequence, and a hGT terminator sequence,
- a second expression cassette comprising in 5' to 3' direction a hCMV promoter, a nucleic acid encoding an antibody heavy chain, a bGH polyA signal sequence, and a hGT terminator sequence,
whereby the first expression cassette and the second expression cassette are arranged unidirectional,
and thereby obtaining a multitude of recombinant mammalian cells,
b) selecting from the multitude of recombinant mammalian cells a (single) stable transfected recombinant mammalian cell, wherein in cell lines obtained by transfection and selection of stable cell clones the combination of bovine growth hormone polyA signal sequence and human gastrin terminator results in an improved antibody expression yield.

2. The method according to claim 1, **characterized in that** the nucleic acid encoding the antibody light chain and/or the nucleic acid encoding the antibody heavy chain comprises at least one intron.

3. The method according to any one of claims 1 to 2, **characterized in that** the nucleic acid encoding the antibody light chain and/or the nucleic acid encoding the antibody heavy chain is cDNA.

4. The method according to any one of claims 1 to 3, **characterized in that** the expression plasmid further comprises a selection marker.

5. The method according to any one of claims 1 to 4, **characterized in that** the expression cassettes and the selection marker are arranged unidirectional.

6. The method according to any one of claims 1 to 5, **characterized in that** the expression cassettes are arranged in the sequence LC-HC-SM.

7. The method according to any one of claims 1 to 6, **characterized in that** the mammalian cell is selected from CHO cell, HEK cell, BHK cell, NS0 cell, and SP2/0 cell.

8. The method according to any one of claims 1 to 7, **characterized in that** the mammalian cell is a CHO cell for the selection of a stable transfected cell.

9. A method for producing an antibody comprising the following steps:
a) cultivating a mammalian cell comprising
- a first expression cassette comprising in 5' to 3' direction a hCMV promoter, a nucleic acid encoding an antibody light chain, a bGH polyA signal sequence, and a hGT terminator sequence,
- a second expression cassette comprising in 5' to 3' direction a hCMV promoter, a nucleic acid encoding an antibody heavy chain, a bGH polyA signal sequence, and a hGT terminator sequence, and
b) recovering the antibody from the cell or the cultivation medium,
whereby the first expression cassette and the second expression cassette are arranged unidirectional for the stable production of the antibody.

10. The method according to claim 9, **characterized in that** the nucleic acid encoding the antibody light chain and/or the nucleic acid encoding the antibody heavy chain comprises at least one intron.

11. The method according to any one of claims 9 to 10, **characterized in that** the nucleic acid encoding the antibody light chain and/or the nucleic acid encoding the antibody heavy chain is cDNA.

12. The method according to any one of claims 9 to 11, **characterized in that** the expression plasmid further comprises a selection marker.

13. The method according to any one of claims 9 to 12, **characterized in that** the expression cassettes and the selection marker are arranged unidirectional.

14. The method according to any one of claims 9 to 13, **characterized in that** the expression cassettes are arranged in the sequence LC-HC-SM.

15. The method according to any one of claims 9 to 14, **characterized in that** the mammalian cell is selected from CHO cell, HEK cell, BHK cell, NS0 cell, and SP2/0 cell.

16. The method according to any one of claims 9 to 15, **characterized in that** the mammalian cell is a CHO cell for the stable production of an antibody.

17. An expression vector comprising
- a first expression cassette comprising in 5' to 3' direction a hCMV promoter, a nucleic acid encoding an antibody light chain, a bGH polyA signal sequence, and a hGT terminator sequence,
- a second expression cassette comprising in 5' to 3' direction a hCMV promoter, a nucleic acid encoding an antibody heavy chain, a bGH polyA signal sequence, and a hGT terminator sequence.

18. The expression vector according to claim 17, **characterized in that** the nucleic acid encoding the antibody light chain and/or the nucleic acid encoding the antibody heavy chain comprises at least one intron.

19. The expression vector according to any one of claims 17 to 18, **characterized in that** the nucleic acid encoding the antibody light chain and/or the nucleic acid encoding the antibody heavy chain is cDNA.

20. The expression vector according to any one of claims 17 to 19, **characterized in that** the first expression cassette and the second expression cassette are arranged unidirectional for the selection of a stable transfected cell.

21. The expression vector according to any one of claims 17 to 20, **characterized in that** the expression plasmid further comprises a selection marker.

22. The expression vector according to any one of claims 17 to 21, **characterized in that** the expression cassettes and the selection marker are arranged unidirectional.

23. The expression vector according to any one of claims 17 to 22, **characterized in that** the expression cassettes are arranged in the sequence LC-HC-SM.

24. A eukaryotic cell comprising the expression vector according to any one of claims 17 to 22.

## Patentansprüche

1. Verfahren zum Auswählen einer rekombinanten stabilen transfizierten Säugerzelle, umfassend die folgenden Schritte:
a) Transfizieren einer Säugerzelle mit einem Expressionsvektor, umfassend
- eine erste Expressionskassette, die in 5'- zu 3'-Richtung einen hCMV-Promotor, eine Nukleinsäure, die für eine leichte Kette eines Antikörpers kodiert, eine bGH-polyA-Signalsequenz und eine hGT-Terminatorsequenz umfasst,
- eine zweite Expressionskassette, die in 5'- zu 3'-Richtung einen hCMV-Promotor, eine Nukleinsäure, die für eine schwere Kette eines Antikörpers kodiert, eine bGH-polyA-Signalsequenz und eine hGT-Terminatorsequenz umfasst,
wobei die erste Expressionskassette und die zweite Expressionskassette unidirektional angeordnet sind,
und dadurch Erhalten einer Vielzahl von rekombinanten Säugerzellen,
b) Auswählen einer (einzelnen) stabilen transfizierten rekombinanten Säugerzelle aus der Vielzahl von rekombinanten Säugerzellen, wobei in Zelllinien, die durch Transfektion und Auswahl stabiler Zellklone erhalten wurden, die Kombination von Rinderwachstumshormon-polyA-Signalsequenz und humanem Gastrin-Terminator zu einer verbesserten Antikörperexpressionsausbeute führt.

2. Verfahren nach Anspruch 1, **dadurch gekennzeichnet, dass** die Nukleinsäure, die für die leichte Kette des Antikörpers kodiert, und/oder die Nukleinsäure, die für die schwere Kette des Antikörpers kodiert, mindestens ein Intron umfassen/umfasst.

3. Verfahren nach einem der Ansprüche 1 bis 2, **dadurch gekennzeichnet, dass** die Nukleinsäure, die für die leichte Kette des Antikörpers kodiert, und/oder die Nukleinsäure, die für die schwere Kette des Antikörpers kodiert, cDNA sind/ist.

4. Verfahren nach einem der Ansprüche 1 bis 3, **dadurch gekennzeichnet, dass** das Expressionsplasmid ferner einen Selektionsmarker umfasst.

5. Verfahren nach einem der Ansprüche 1 bis 4, **dadurch gekennzeichnet, dass** die Expressionskassetten und der Selektionsmarker unidirektional angeordnet sind.

6. Verfahren nach einem der Ansprüche 1 bis 5, **dadurch gekennzeichnet, dass** die Expressionskassetten in der Reihenfolge LC-HC-SM angeordnet sind.

7. Verfahren nach einem der Ansprüche 1 bis 6, **dadurch gekennzeichnet, dass** die Säugerzelle aus einer CHO-Zelle, HEK-Zelle, BHK-Zelle, NS0-Zelle und SP2/0-Zelle ausgewählt ist.

8. Verfahren nach einem der Ansprüche 1 bis 7, **dadurch gekennzeichnet, dass** die Säugerzelle eine CHO-Zelle für die Auswahl einer stabilen transfizierten Zelle ist.

9. Verfahren zum Produzieren eines Antikörpers, umfassend die folgenden Schritte:
a) Kultivieren einer Säugerzelle, umfassend
- eine erste Expressionskassette, die in 5'- zu 3'-Richtung einen hCMV-Promotor, eine Nukleinsäure, die für eine leichte Kette eines Antikörpers kodiert, eine bGH-polyA-Signalsequenz und eine hGT-Terminatorsequenz umfasst,
- eine zweite Expressionskassette, die in 5'- zu 3'-Richtung einen hCMV-Promotor, eine Nukleinsäure, die für eine schwere Kette eines Antikörpers kodiert, eine bGH-polyA-Signalsequenz und eine hGT-Terminatorsequenz umfasst, und
b) Gewinnen des Antikörpers aus der Zelle oder dem Kultivierungsmedium,
wobei die erste Expressionskassette und die zweite Expressionskassette für die stabile Produktion des Antikörpers unidirektional angeordnet sind.

10. Verfahren nach Anspruch 9, **dadurch gekennzeichnet, dass** die Nukleinsäure, die für die leichte Kette des Antikörpers kodiert, und/oder die Nukleinsäure, die für die schwere Kette des Antikörpers kodiert, mindestens ein Intron umfassen/umfasst.

11. Verfahren nach einem der Ansprüche 9 bis 10, **dadurch gekennzeichnet, dass** die Nukleinsäure, die für die leichte Kette des Antikörpers kodiert, und/oder die Nukleinsäure, die für die schwere Kette des Antikörpers kodiert, cDNA sind/ist.

12. Verfahren nach einem der Ansprüche 9 bis 11, **dadurch gekennzeichnet, dass** das Expressionsplasmid ferner einen Selektionsmarker umfasst.

13. Verfahren nach einem der Ansprüche 9 bis 12, **dadurch gekennzeichnet, dass** die Expressionskassetten und der Selektionsmarker unidirektional angeordnet sind.

14. Verfahren nach einem der Ansprüche 9 bis 13, **dadurch gekennzeichnet, dass** die Expressionskassetten in der Reihenfolge LC-HC-SM angeordnet sind

15. Verfahren nach einem der Ansprüche 9 bis 14, **dadurch gekennzeichnet, dass** die Säugerzelle aus einer CHO-Zelle, HEK-Zelle, BHK-Zelle, NS0-Zelle und SP2/0-Zelle ausgewählt ist.

16. Verfahren nach einem der Ansprüche 9 bis 15, **dadurch gekennzeichnet, dass** die Säugerzelle eine CHO-Zelle für die stabile Produktion eines Antikörpers ist.

17. Expressionsvektor, umfassend
- eine erste Expressionskassette, die in 5'- zu 3'-Richtung einen hCMV-Promotor, eine Nukleinsäure, die für eine leichte Kette eines Antikörpers kodiert, eine bGH-polyA-Signalsequenz und eine hGT-Terminatorsequenz umfasst,
- eine zweite Expressionskassette, die in 5'- zu 3'-Richtung einen hCMV-Promotor, eine Nukleinsäure, die für eine schwere Kette eines Antikörpers kodiert, eine bGH-polyA-Signalsequenz und eine hGT-Terminatorsequenz umfasst.

18. Expressionsvektor nach Anspruch 17, **dadurch gekennzeichnet, dass** die Nukleinsäure, die für die leichte Kette des Antikörpers kodiert, und/oder die Nukleinsäure, die für die schwere Kette des Antikörpers kodiert, mindestens ein Intron umfassen/umfasst.

19. Expressionsvektor nach einem der Ansprüche 17 bis 18, **dadurch gekennzeichnet, dass** die Nukleinsäure, die für die leichte Kette des Antikörpers kodiert, und/oder die Nukleinsäure, die für die schwere Kette des Antikörpers kodiert, cDNA sind/ist.

20. Expressionsvektor nach einem der Ansprüche 17 bis 19, **dadurch gekennzeichnet, dass** die erste Expressionskassette und die zweite Expressionskassette für die Auswahl einer stabilen transfizierten Zelle unidirektional angeordnet sind.

21. Expressionsvektor nach einem der Ansprüche 17 bis 20, **dadurch gekennzeichnet, dass** das Expressionsplasmid ferner einen Selektionsmarker umfasst.

22. Expressionsvektor nach einem der Ansprüche 17 bis 21, **dadurch gekennzeichnet, dass** die Expressionskassetten und der Selektionsmarker unidirektional angeordnet sind.

23. Expressionsvektor nach einem der Ansprüche 17 bis 22, **dadurch gekennzeichnet, dass** die Expressionskassetten in der Reihenfolge LC-HC-SM angeordnet sind.

24. Eukaryotische Zelle, umfassend den Expressionsvektor nach einem der Ansprüche 17 bis 22.

## Revendications

1. Procédé de sélection d'une cellule de mammifère transfectée stable recombinante comprenant les étapes suivantes :
a) transfection d'une cellule de mammifère avec un vecteur d'expression comprenant
- une première cassette d'expression comprenant dans la direction 5' à 3' un promoteur de hCMV, un acide nucléique codant pour une chaîne légère d'anticorps, une séquence signal polyA de bGH et une séquence terminateur de hGT,
- une seconde cassette d'expression comprenant dans la direction 5' à 3' un promoteur de hCMV, un acide nucléique codant pour une chaîne lourde d'anticorps, une séquence signal polyA de bGH et une séquence terminateur de hGT,
moyennant quoi la première cassette d'expression et la seconde cassette d'expression sont agencées de manière unidirectionnelle,
et obtenant ainsi une multitude de cellules de mammifères recombinantes,
b) sélection, parmi la multitude de cellules de mammifères recombinantes, d'une (seule) cellule de mammifère recombinante transfectée stable, dans lequel, dans les lignées cellulaires obtenues par transfection et sélection de clones de cellules stables, la combinaison de la séquence signal polyA d'hormone de croissance bovine et du terminateur de gastrine humaine se traduit par une amélioration du rendement d'expression d'anticorps.

2. Procédé selon la revendication 1, **caractérisé en ce que** l'acide nucléique codant pour la chaîne légère d'anticorps et/ou l'acide nucléique codant pour la chaîne lourde d'anticorps comprennd au moins un intron.

3. Procédé selon l'une quelconque des revendications 1 à 2, **caractérisé en ce que** l'acide nucléique codant pour la chaîne légère d'anticorps et/ou l'acide nucléique codant pour la chaîne lourde d'anticorps est de l'ADNc.

4. Procédé selon l'une quelconque des revendications 1 à 3, **caractérisé en ce que** le plasmide d'expression comprend en outre un marqueur de sélection.

5. Procédé selon l'une quelconque des revendications 1 à 4, **caractérisé en ce que** les cassettes d'expression et le marqueur de sélection sont agencés de manière unidirectionnelle.

6. Procédé selon l'une quelconque des revendications 1 à 5, **caractérisé en ce que** les cassettes d'expression sont agencées dans la séquence LC-HC-SM.

7. Procédé selon l'une quelconque des revendications 1 à 6, **caractérisé en ce que** la cellule de mammifère est sélectionnée parmi une cellule CHO, une cellule HEK, une cellule BHK, une cellule NS0 et une cellule SP2/0.

8. Procédé selon l'une quelconque des revendications 1 à 7, **caractérisé en ce que** la cellule de mammifère est une cellule CHO pour la sélection d'une cellule transfectée stable.

9. Procédé de production d'un anticorps comprenant les étapes suivantes :
a) culture d'une cellule de mammifère comprenant
- une première cassette d'expression comprenant dans la direction 5' à 3' un promoteur de hCMV, un acide nucléique codant pour une chaîne légère d'anticorps, une séquence signal polyA de bGH et une séquence terminateur de hGT,
- une seconde cassette d'expression comprenant dans la direction 5' à 3' un promoteur de hCMV, un acide nucléique codant pour une chaîne lourde d'anticorps, une séquence signal polyA de bGH et une séquence terminateur de hGT, et
b) récupération de l'anticorps à partir de la cellule ou du milieu de culture,
moyennant quoi la première cassette d'expression et la seconde cassette d'expression sont agencées de manière unidirectionnelle pour la production stable de l'anticorps.

10. Procédé selon la revendication 9, **caractérisé en ce que** l'acide nucléique codant pour la chaîne légère d'anticorps et/ou l'acide nucléique codant pour la chaîne lourde d'anticorps comprennd au moins un intron.

11. Procédé selon l'une quelconque des revendications 9 à 10, **caractérisé en ce que** l'acide nucléique codant pour la chaîne légère d'anticorps et/ou l'acide nucléique codant pour la chaîne lourde d'anticorps est de l'ADNc.

12. Procédé selon l'une quelconque des revendications 9 à 11, **caractérisé en ce que** le plasmide d'expression comprend en outre un marqueur de sélection.

13. Procédé selon l'une quelconque des revendications 9 à 12, **caractérisé en ce que** les cassettes d'expression et le marqueur de sélection sont agencés de manière unidirectionnelle.

14. Procédé selon l'une quelconque des revendications 9 à 13, **caractérisé en ce que** les cassettes d'expression sont agencées dans la séquence LC-HC-SM.

15. Procédé selon l'une quelconque des revendications 9 à 14, **caractérisé en ce que** la cellule de mammifère est sélectionnée parmi une cellule CHO, une cellule HEK, une cellule BHK, une cellule NS0 et une cellule SP2/0.

16. Procédé selon l'une quelconque des revendications 9 à 15, **caractérisé en ce que** la cellule de mammifère est une cellule CHO pour la production stable d'un anticorps.

17. Vecteur d'expression comprenant
- une première cassette d'expression comprenant dans la direction 5' à 3' un promoteur de hCMV, un acide nucléique codant pour une chaîne légère d'anticorps, une séquence signal polyA de bGH et une séquence terminateur de hGT,
- une seconde cassette d'expression comprenant dans la direction 5' à 3' un promoteur de hCMV, un acide nucléique codant pour une chaîne lourde d'anticorps, une séquence signal polyA de bGH et une séquence terminateur de hGT.

18. Vecteur d'expression selon la revendication 17, **caractérisé en ce que** l'acide nucléique codant pour la chaîne légère d'anticorps et/ou l'acide nucléique codant pour la chaîne lourde d'anticorps comprennd au moins un intron.

19. Vecteur d'expression selon l'une quelconque des revendications 17 à 18, **caractérisé en ce que** l'acide nucléique codant pour la chaîne légère d'anticorps et/ou l'acide nucléique codant pour la chaîne lourde d'anticorps est de l'ADNc.

20. Vecteur d'expression selon l'une quelconque des revendications 17 à 19, **caractérisé en ce que** la première cassette d'expression et la seconde cassette d'expression sont agencées de manière unidirectionnelle pour la sélection d'une cellule transfectée stable.

21. Vecteur d'expression selon l'une quelconque des revendications 17 à 20, **caractérisé en ce que** le plasmide d'expression comprend en outre un marqueur de sélection.

22. Vecteur d'expression selon l'une quelconque des revendications 17 à 21, **caractérisé en ce que** les cassettes d'expression et le marqueur de sélection sont agencés de manière unidirectionnelle.

23. Vecteur d'expression selon l'une quelconque des revendications 17 à 22, **caractérisé en ce que** les cassettes d'expression sont agencées dans la séquence LC-HC-SM.

24. Cellule eucaryote comprenant le vecteur d'expression selon l'une quelconque des revendications 17 à 22.


### SEQUENCE LISTING

<110> F. Hoffmann-La Roche AG
<120> Expression vector element combinations, novel production cell generation methods and their use for the recombinant production of polypeptides
<130> 30789 WO
<150> EP11195361
   <151> 2011-12-22
<160> 17
<170> PatentIn version 3.5
<210> 1
   <211> 608
   <212> DNA
   <213> Human cytomegalovirus
<400> 1
   gttgacattg attattgact agttattaat agtaatcaat tacggggtca ttagttcata        60
   gcccatatat ggagttccgc gttacataac ttacggtaaa tggcccgcct ggctgaccgc       120
   ccaacgaccc ccgcccattg acgtcaataa tgacgtatgt tcccatagta acgccaatag       180
   ggactttcca ttgacgtcaa tgggtggagt atttacggta aactgcccac ttggcagtac       240
   atcaagtgta tcatatgcca agtacgcccc ctattgacgt caatgacggt aaatggcccg       300
   cctggcatta tgcccagtac atgaccttat gggactttcc tacttggcag tacatctacg       360
   tattagtcat cgctattagc atggtgatgc ggttttggca gtacatcaat gggcgtggat       420
   agcggtttga ctcacgggga tttccaagtc tccaccccat tgacgtcaat gggagtttgt       480
   tttggcacca aaatcaacgg gactttccaa aatgtcgtaa caactccgcc ccattgacgc       540
   aaatgggcgg taggcgtgta cggtgggagg tctatataag cagagctccg tttagtgaac       600
   gtcagatc                                                                608
<210> 2
   <211> 696
   <212> DNA
   <213> Human cytomegalovirus
<400> 2
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtacgcccc ctattgacgt caatgacggt aaatggcccg 300
   cctggcatta tgcccagtac atgaccttat gggactttcc tacttggcag tacatctacg 360
   tattagtcat cgctattagc atggtgatgc ggttttggca gtacatcaat gggcgtggat 420
   agcggtttga ctcacgggga tttccaagtc tccaccccat tgacgtcaat gggagtttgt 480
   tttggcacca aaatcaacgg gactttccaa aatgtcgtaa caactccgcc ccattgacgc 540
   aaatgggcgg taggcgtgta cggtgggagg tctatataag cagagctccg tttagtgaac 600
   gtcagatcta gctctgggag aggagcccag cactagaagt cggcggtgtt tccattcggt 660
   gatcagcact gaacacagag gaagcttgcc gccacc 696
<210> 3
   <211> 2125
   <212> DNA
   <213> Human cytomegalovirus
<400> 3
   ctgcagtgaa taataaaatg tgtgtttgtc cgaaatacgc gttttgagat ttctgtcgcc      60
   gactaaattc atgtcgcgcg atagtggtgt ttatcgccga tagagatggc gatattggaa     120
   aaatcgatat ttgaaaatat ggcatattga aaatgtcgcc gatgtgagtt tctgtgtaac     180
   tgatatcgcc atttttccaa aagtgatttt tgggcatacg cgatatctgg cgatagcgct     240
   tatatcgttt acgggggatg gcgatagacg actttggtga cttgggcgat tctgtgtgtc     300
   gcaaatatcg cagtttcgat ataggtgaca gacgatatga ggctatatcg ccgatagagg     360
   cgacatcaag ctggcacatg gccaatgcat atcgatctat acattgaatc aatattggcc     420
   attagccata ttattcattg gttatatagc ataaatcaat attggctatt ggccattgca     480
   tacgttgtat ccatatcata atatgtacat ttatattggc tcatgtccaa cattaccgcc     540
   atgttgacat tgattattga ctagttatta atagtaatca attacggggt cattagttca     600
   tagcccatat atggagttcc gcgttacata acttacggta aatggcccgc ctggctgacc     660
   gcccaacgac ccccgcccat tgacgtcaat aatgacgtat gttcccatag taacgccaat     720
   agggactttc cattgacgtc aatgggtgga gtatttacgg taaactgccc acttggcagt     780
   acatcaagtg tatcatatgc caagtacgcc ccctattgac gtcaatgacg gtaaatggcc     840
   cgcctggcat tatgcccagt acatgacctt atgggacttt cctacttggc agtacatcta     900
   cgtattagtc atcgctatta ccatggtgat gcggttttgg cagtacatca atgggcgtgg     960
   atagcggttt gactcacggg gatttccaag tctccacccc attgacgtca atgggagttt    1020
   gttttggcac caaaatcaac gggactttcc aaaatgtcgt aacaactccg ccccattgac    1080
   gcaaatgggc ggtaggcgtg tacggtggga ggtctatata agcagagctc gtttagtgaa    1140
   ccgtcagatc gcctggagac gccatccacg ctgttttgac ctccatagaa gacaccggga    1200
   ccgatccagc ctccgcggcc gggaacggtg cattggaacg cggattcccc gtgccaagag    1260
   tgacgtaagt accgcctata gagtctatag gcccaccccc ttggcttctt atgcatgcta    1320
   tactgttttt ggcttggggt ctatacaccc ccgcttcctc atgttatagg tgatggtata 1380
   gcttagccta taggtgtggg ttattgacca ttattgacca ctcccctatt ggtgacgata 1440
   ctttccatta ctaatccata acatggctct ttgccacaac tctctttatt ggctatatgc 1500
   caatacactg tccttcagag actgacacgg actctgtatt tttacaggat ggggtctcat 1560
   ttattattta caaattcaca tatacaacac caccgtcccc agtgcccgca gtttttatta 1620
   aacataacgt gggatctcca cgcgaatctc gggtacgtgt tccggacatg ggctcttctc 1680
   cggtagcggc ggagcttcta catccgagcc ctgctcccat gcctccagcg actcatggtc 1740
   gctcggcagc tccttgctcc taacagtgga ggccagactt aggcacagca cgatgcccac 1800
   caccaccagt gtgccgcaca aggccgtggc ggtagggtat gtgtctgaaa atgagctcgg 1860
   ggagcgggct tgcaccgctg acgcatttgg aagacttaag gcagcggcag aagaagatgc 1920
   aggcagctga gttgttgtgt tctgataaga gtcagaggta actcccgttg cggtgctgtt 1980
   aacggtggag ggcagtgtag tctgagcagt actcgttgct gccgcgcgcg ccaccagaca 2040
   taatagctga cagactaaca gactgttcct ttccatgggt cttttctgca gtcaccgtcc 2100
   ttgacacggt ttaaacgccg ccacc 2125
<210> 4
   <211> 575
   <212> **DNA**
   <213> Homo sapiens
<400> 4
   cccgggctgg gctgagaccc gcagaggaag acgctctagg gatttgtccc ggactagcga 60
   gatggcaagg ctgaggacgg gaggctgatt gagaggcgaa ggtacaccct aatctcaata 120
   caacctttgg agctaagcca gcaatggtag agggaagatt ctgcacgtcc cttccaggcg 180
   gcctccccgt caccaccccc cccaacccgc cccgaccgga gctgagagta attcatacaa 240
   aaggactcgc ccctgccttg gggaatccca gggaccgtcg ttaaactccc actaacgtag 300
   aacccagaga tcgctgcgtt cccgccccct cacccgcccg ctctcgtcat cactgaggtg 360
   gagaagagca tgcgtgaggc tccggtgccc gtcagtgggc agagcgcaca tcgcccacag 420
   tccccgagaa gttgggggga ggggtcggca attgaaccgg tgcctagaga aggtggcgcg 480
   gggtaaactg ggaaagtgat gtcgtgtact ggctccgcct ttttcccgag ggtgggggag 540
   aaccgtatat aagtgcagta gtcgccgtga acgtt 575
<210> 5
   <211> 1571
   <212> DNA
   <213> Homo sapiens
<400> 5
   cccgggctgg gctgagaccc gcagaggaag acgctctagg gatttgtccc ggactagcga 60
   gatggcaagg ctgaggacgg gaggctgatt gagaggcgaa ggtacaccct aatctcaata 120
   caacctttgg agctaagcca gcaatggtag agggaagatt ctgcacgtcc cttccaggcg 180
   gcctccccgt caccaccccc cccaacccgc cccgaccgga gctgagagta attcatacaa 240
   aaggactcgc ccctgccttg gggaatccca gggaccgtcg ttaaactccc actaacgtag 300
   aacccagaga tcgctgcgtt cccgccccct cacccgcccg ctctcgtcat cactgaggtg 360
   gagaagagca tgcgtgaggc tccggtgccc gtcagtgggc agagcgcaca tcgcccacag 420
   tccccgagaa gttgggggga ggggtcggca attgaaccgg tgcctagaga aggtggcgcg 480
   gggtaaactg ggaaagtgat gtcgtgtact ggctccgcct ttttcccgag ggtgggggag 540
   aaccgtatat aagtgcagta gtcgccgtga acgttctttt tcgcaacggg tttgccgcca 600
   gaacacaggt aagtgccgtg tgtggttccc gcgggcctgg cctctttacg ggttatggcc 660
   cttgcgtgcc ttgaattact tccacgcccc tggctgcagt acgtgattct tgatcccgag 720
   cttcgggttg gaagtgggtg ggagagttcg aggccttgcg cttaaggagc cccttcgcct 780
   cgtgcttgag ttgaggcctg gcctgggcgc tggggccgcc gcgtgcgaat ctggtggcac 840
   cttcgcgcct gtctcgctgc tttcgataag tctctagcca tttaaaattt ttgatgacct 900
   gctgcgacgc tttttttctg gcaagatagt cttgtaaatg cgggccaaga tctgcacact 960
   ggtatttcgg tttttggggc cgcgggcggc gacggggccc gtgcgtccca gcgcacatgt 1020
   tcggcgaggc ggggcctgcg agcgcggcca ccgagaatcg gacgggggta gtctcaagct 1080
   ggccggcctg ctctggtgcc tggcctcgcg ccgccgtgta tcgccccgcc ctgggcggca 1140
   aggctggccc ggtcggcacc agttgcgtga gcggaaagat ggccgcttcc cggccctgct 1200
   gcagggagct caaaatggag gacgcggcgc tcgggagagc gggcgggtga gtcacccaca 1260
   caaaggaaaa gggcctttcc gtcctcagcc gtcgcttcat gtgactccac ggagtaccgg 1320
   gcgccgtcca ggcacctcga ttagttctcg atcttttgga gtacgtcgtc tttaggttgg 1380
   ggggaggggt tttatgcgat ggagtttccc cacactgagt gggtggagac tgaagttagg 1440
   ccagcttggc acttgatgta attctccttg gaatttgccc tttttgagtt tggatcttgg 1500
   ttcattctca agcctcagac agtggttcaa agtttttttc ttccatttca ggtggtttaa 1560
   acgccgccac c 1571
<210> 6
   <211> 1653
   <212> DNA
   <213> Artificial Sequence
<220>
   <223> human elongation factor 1 alpha promoter with Intron A and with optimized 5'UTR
<400> 6
   cccgggctgg gctgagaccc gcagaggaag acgctctagg gatttgtccc ggactagcga      60
   gatggcaagg ctgaggacgg gaggctgatt gagaggcgaa ggtacaccct aatctcaata     120
   caacctttgg agctaagcca gcaatggtag agggaagatt ctgcacgtcc cttccaggcg     180
   gcctccccgt caccaccccc cccaacccgc cccgaccgga gctgagagta attcatacaa     240
   aaggactcgc ccctgccttg gggaatccca gggaccgtcg ttaaactccc actaacgtag     300
   aacccagaga tcgctgcgtt cccgccccct cacccgcccg ctctcgtcat cactgaggtg     360
   gagaagagca tgcgtgaggc tccggtgccc gtcagtgggc agagcgcaca tcgcccacag     420
   tccccgagaa gttgggggga ggggtcggca attgaaccgg tgcctagaga aggtggcgcg     480
   gggtaaactg ggaaagtgat gtcgtgtact ggctccgcct ttttcccgag ggtgggggag     540
   aaccgtatat aagtgcagta gtcgccgtga acgttctttt tcgcaacggg tttgccgcca     600
   gaacacaggt aagtgccgtg tgtggttccc gcgggcctgg cctctttacg ggttatggcc     660
   cttgcgtgcc ttgaattact tccacgcccc tggctgcagt acgtgattct tgatcccgag     720
   cttcgggttg gaagtgggtg ggagagttcg aggccttgcg cttaaggagc cccttcgcct     780
   cgtgcttgag ttgaggcctg gcctgggcgc tggggccgcc gcgtgcgaat ctggtggcac     840
   cttcgcgcct gtctcgctgc tttcgataag tctctagcca tttaaaattt ttgatgacct     900
   gctgcgacgc tttttttctg gcaagatagt cttgtaaatg cgggccaaga tctgcacact     960
   ggtatttcgg tttttggggc cgcgggcggc gacggggccc gtgcgtccca gcgcacatgt    1020
   tcggcgaggc ggggcctgcg agcgcggcca ccgagaatcg gacgggggta gtctcaagct    1080
   ggccggcctg ctctggtgcc tggcctcgcg ccgccgtgta tcgccccgcc ctgggcggca    1140
   aggctggccc ggtcggcacc agttgcgtga gcggaaagat ggccgcttcc cggccctgct    1200
   gcagggagct caaaatggag gacgcggcgc tcgggagagc gggcgggtga gtcacccaca    1260
   caaaggaaaa gggcctttcc gtcctcagcc gtcgcttcat gtgactccac ggagtaccgg    1320
   gcgccgtcca ggcacctcga ttagttctcg atcttttgga gtacgtcgtc tttaggttgg    1380
   ggggaggggt tttatgcgat ggagtttccc cacactgagt gggtggagac tgaagttagg    1440
   ccagcttggc acttgatgta attctccttg gaatttgccc tttttgagtt tggatcttgg    1500
   ttcattctca agcctcagac agtggttcaa agtttttttc ttccatttca ggtgtcgtga    1560
   ggaattagct ctgggagagg agcccagcac tagaagtcgg cggtgtttcc attcggtgat    1620
   cagcactgaa cacagaggaa gcttgccgcc acc                                 1653
<210> 7
   <211> 2473
   <212> **DNA**
   <213> Rattus norvegicus
<400> 7
   gatattttta tggaaatttt aaaaaattct ggtaagctat ttaaaaaaat gaactttatt 60
   atgaaactat tgcccttttc tctaaaaaac aacacaattt cacggaatat cctatgatta 120
   attatgacct tttagccagt tcccatatta agaatgagtt atagatgact ctctttaaaa 180
   aattattcga tttaaaccat ctgttttaaa gcacagcatt tgtgaataat gtgaagaact 240
   tagaagtata atctactcca aggtctgatg tatttttcaa ggccacgtta aagtgtatgc 300
   ttgtaacaga gtgcttacat tcaagccaaa tgttaatata acaatcctga attcgtacat 360
   aatgtgaata agacactcaa ctctatttaa atccagatct aaatagttac ttttatctaa 420
   atgtcaccat ctgtttctac ttagaataat aaacttctta aaggtcacgt atcgggctga 480
   ttataaatca ttataattat aacaaaacag atgatttgtt taaaggtcac atcccgttcc 540
   gtggtctttt tagtcgaaat aactattaat cttcattatg tttctgagaa agtttaaata 600
   tcacgatttc cacccataac agtcattatg agtcagtggg agtcatactg aatcagggta 660
   ttttaactgg aaattttttg aaaaacatga gtttttctta aggtcaacat ctggtcttat 720
   aaacagaact gagatttatg gccggtaatt accactggac gatttcccgg gaaatcgcta 780
   tgggaacggc ccgttttgca acttctttga ccaaaatata tcgagttaag caacttttaa 840
   ggccaagtca ctatgactat gccaaataaa gcaactatta aggtcatttc actatggaaa 900
   cacccaattc agcaacattg taagccaaat ctccatagaa acctcataag tcagccaaaa 960
   gtcaacgacc taccatctgt ttctgcttat ttctctaatt ttaattgcag actttgtcat 1020
   tttatgttcc tcttattctg agaatacgtg acgcccgctc gttaaggaca ccgaaactgc 1080
   ataagagtca cgttgactca gatgacctcg acatctggtc tggtttttct gccaattttt 1140
   cgtctaaact gtggaaaatc cccacagatg acctacaaaa ctccgatttc tattggacga 1200
   tgaccgtcag acgtaggtat aaatctccta acgccgttcg ggcagtcaca gtcttcggat 1260
   cggacgccgt ggaacgcagt tctcagcgaa gaaggacacc gcccgactcc agaagacacc 1320
   gctgcccgaa gaagagaaga cttcatcggt aagagaccca gcttctcctc cccggagctt 1380
   cggccacgcc gctccacacc cgggaaccga ggcttcggag cccgataccc ggacagaagc 1440
   ttctccccgg ccgctccaca tcagggagcc ttgaccggcg agcctgctat ccgggtagag 1500
   actgtcctgc ggccgcttca gcagctccac gatcgacgac tgtgaccgtt gagcccgccg 1560
   tttaggcaga ggctccgctt caactaccct accgacacat tcgcggttct tcctccagaa 1620
   catcttaccc tctactcggc cactctacaa ggaccggtaa gcaattttta tatactagac 1680
   ttaaatgttt ctatgatcat tatgtggtga tggttctgtg tatgaagaga gctaggtgga 1740
   ggctatcttt cgcttcggtg atggaacact actcttacaa tggcggctct aatgacggtt 1800
   ttctcaacat cggtggcggc tctaattacg gttctctcaa catcggtggt ggtcttcgca 1860
   tgcgagctct agattttttt tatctgtaaa ataagattga agatggttga ctgtgtatca 1920
   attctttttc ataggcatca gatcttgtca accgttatta atctttagga tcagatgaac 1980
   ttgcgagctc gatatctaga atagaatccc cgtgactgct aagatcatct ccgttcatac 2040
   accagatgtt acaggccacg gctaccatta tgaatccaaa catgaacaga attgccagaa 2100
   tggtgctcaa tggttgtatc catctcgctg gtctattttc tctcaccgac gagaccccaa 2160
   catcgagagt tccgtttatt tcatgagtcg accttttagt tcgtgattta ttttctgtgt 2220
   taagaaaatc agtgagatca attattgtca gtctatacga ttacaataat gtctgaatta 2280
   tcgacgtgca taagatcgtc tcacccggcg cagattccaa cagatctttg tcgccatgcc 2340
   ttccgttaga aaggtagtat agtaatatga taccagcaat gcacagaatc gaacatttga 2400
   taacaatttt gttgatgtcg tatatctgtt aaaaattaat aaatatatta cagtcagttt 2460
   aaacgccgcc acc 2473
<210> 8
   <211> 129
   <212> DNA
   <213> Simian virus 40
<400> 8
   aacttgttta ttgcagctta taatggttac aaataaagca atagcatcac aaatttcaca 60
   aataaagcat ttttttcacc attctagttg tggtttgtcc aaactcatca atgtatctta 120
   tcatgtctg 129
<210> 9
   <211> 225
   <212> DNA
   <213> Bos taurus
<400> 9
   ctgtgccttc tagttgccag ccatctgttg tttgcccctc ccccgtgcct tccttgaccc 60
   tggaaggtgc cactcccact gtcctttcct aataaaatga ggaaattgca tcgcattgtc 120
   tgagtaggtg tcattctatt ctggggggtg gggtggggca ggacagcaag ggggaggatt 180
   gggaagacaa tagcaggcat gctggggatg cggtgggctc tatgg 225
<210> 10
   <211> 73
   <212> DNA
   <213> Homo sapiens
<400> 10
   caggataata tatggtaggg ttcatagcca gagtaacctt tttttttaat ttttatttta 60
   ttttattttt gag 73
<210> 11
   <211> 288
   <212> DNA
   <213> Simian virus 40
<400> 11
   agtcagcaac caggtgtgga aagtccccag gctccccagc aggcagaagt atgcaaagca 60
   tgcatctcaa ttagtcagca accatagtcc cgcccctaac tccgcccatc ccgcccctaa 120
   ctccgcccag ttccgcccat tctccgcccc atggctgact aatttttttt atttatgcag 180
   aggccgaggc cgcctctgcc tctgagctat tccagaagta gtgaggaggc ttttttggag 240
   gcctaggctt ttgcaaaaag ctcccgggag cttgtatatc cattttcg 288
<210> 12
   <211> 81
   <212> DNA
   <213> Homo sapiens
<400> 12
   catggcttcc cgccggaggt ggaggagcag gatgatggca cgctgcccat gtcttgtgcc 60
   caggagagcg ggatggaccg t 81
<210> 13
   <211> 798
   <212> DNA
   <213> Artificial Sequence
<220>
   <223> green fluorescent protein encoding nucleic acid
<400> 13
   atggtgagca agggcgagga gctgttcacc ggggtggtgc ccatcctggt cgagctggac      60
   ggcgacgtaa acggccacaa gttcagcgtg tccggcgagg gcgagggcga tgccacctac     120
   ggcaagctga ccctgaagtt catctgcacc accggcaagc tgcccgtgcc ctggcccacc     180
   ctcgtgacca ccctgaccta cggcgtgcag tgcttcagcc gctaccccga ccacatgaag     240
   cagcacgact tcttcaagtc cgccatgccc gaaggctacg tccaggagcg caccatcttc     300
   ttcaaggacg acggcaacta caagacccgc gccgaggtga agttcgaggg cgacaccctg     360
   gtgaaccgca tcgagctgaa gggcatcgac ttcaaggagg acggcaacat cctggggcac     420
   aagctggagt acaactacaa cagccacaac gtctatatca tggccgacaa gcagaagaac     480
   ggcatcaagg tgaacttcaa gatccgccac aacatcgagg acggcagcgt gcagctcgcc     540
   gaccactacc agcagaacac ccccatcggc gacggccccg tgctgctgcc cgacaaccac     600
   tacctgagca cccagtccgc cctgagcaaa gaccccaacg agaagcgcga tcacatggtc     660
   ctgctggagt tcgtgaccgc cgccgggatc actctcggca tggacgagct gtacaagtcc     720
   ggactcagat ctcgagctca agcttcgaat tctgcagtcg acggtaccgc gggcccggga     780
   tccaccggat ctagatga                                                   798
<210> 14
   <211> 795
   <212> DNA
   <213> Artificial Sequence
<220>
   <223> neomycin selection marker
<400> 14
   atgattgaac aagatggatt gcacgcaggt tctccggccg cttgggtgga gaggctattc 60
   ggctatgact gggcacaaca gacaatcggc tgctctgatg ccgccgtgtt ccggctgtca 120
   gcgcaggggc gcccggttct ttttgtcaag accgacctgt ccggtgccct gaatgaactg 180
   caggacgagg cagcgcggct atcgtggctg gccacgacgg gcgttccttg cgcagctgtg 240
   ctcgacgttg tcactgaagc gggaagggac tggctgctat tgggcgaagt gccggggcag 300
   gatctcctgt catctcacct tgctcctgcc gagaaagtat ccatcatggc tgatgcaatg 360
   cggcggctgc atacgcttga tccggctacc tgcccattcg accaccaagc gaaacatcgc 420
   atcgagcgag cacgtactcg gatggaagcc ggtcttgtcg atcaggatga tctggacgaa 480
   gagcatcagg ggctcgcgcc agccgaactg ttcgccaggc tcaaggcgcg catgcccgac 540
   ggcgaggatc tcgtcgtgac ccatggcgat gcctgcttgc cgaatatcat ggtggaaaat 600
   ggccgctttt ctggattcat cgactgtggc cggctgggtg tggcggaccg ctatcaggac 660
   atagcgttgg ctacccgtga tattgctgaa gagcttggcg gcgaatgggc tgaccgcttc 720
   ctcgtgcttt acggtatcgc cgctcccgat tcgcagcgca tcgccttcta tcgccttctt 780
   gacgagttct tctga 795
<210> 15
   <211> 1677
   <212> DNA
   <213> Artificial Sequence
<220>
   <223> GFP-PEST-NEO fusion polypeptide encoding nucleic acid
<400> 15
   atggtgagca agggcgagga gctgttcacc ggggtggtgc ccatcctggt cgagctggac 60
   ggcgacgtaa acggccacaa gttcagcgtg tccggcgagg gcgagggcga tgccacctac 120
   ggcaagctga ccctgaagtt catctgcacc accggcaagc tgcccgtgcc ctggcccacc 180
   ctcgtgacca ccctgaccta cggcgtgcag tgcttcagcc gctaccccga ccacatgaag 240
   cagcacgact tcttcaagtc cgccatgccc gaaggctacg tccaggagcg caccatcttc 300
   ttcaaggacg acggcaacta caagacccgc gccgaggtga agttcgaggg cgacaccctg 360
   gtgaaccgca tcgagctgaa gggcatcgac ttcaaggagg acggcaacat cctggggcac 420
   aagctggagt acaactacaa cagccacaac gtctatatca tggccgacaa gcagaagaac     480
   ggcatcaagg tgaacttcaa gatccgccac aacatcgagg acggcagcgt gcagctcgcc     540
   gaccactacc agcagaacac ccccatcggc gacggccccg tgctgctgcc cgacaaccac     600
   tacctgagca cccagtccgc cctgagcaaa gaccccaacg agaagcgcga tcacatggtc     660
   ctgctggagt tcgtgaccgc cgccgggatc actctcggca tggacgagct gtacaagtcc     720
   ggactcagat ctcgagctca agcttcgaat tctgcagtcg acggtaccgc gggcccggga     780
   tccaccggat ctagacatgg cttcccgccg gaggtggagg agcaggatga tggcacgctg     840
   cccatgtctt gtgcccagga gagcgggatg gaccgtagtt taaacattga acaagatgga     900
   ttgcacgcag gttctccggc cgcttgggtg gagaggctat tcggctatga ctgggcacaa     960
   cagacaatcg gctgctctga tgccgccgtg ttccggctgt cagcgcaggg gcgcccggtt    1020
   ctttttgtca agaccgacct gtccggtgcc ctgaatgaac tgcaggacga ggcagcgcgg    1080
   ctatcgtggc tggccacgac gggcgttcct tgcgcagctg tgctcgacgt tgtcactgaa    1140
   gcgggaaggg actggctgct attgggcgaa gtgccggggc aggatctcct gtcatctcac    1200
   cttgctcctg ccgagaaagt atccatcatg gctgatgcaa tgcggcggct gcatacgctt    1260
   gatccggcta cctgcccatt cgaccaccaa gcgaaacatc gcatcgagcg agcacgtact    1320
   cggatggaag ccggtcttgt cgatcaggat gatctggacg aagagcatca ggggctcgcg    1380
   ccagccgaac tgttcgccag gctcaaggcg cgcatgcccg acggcgagga tctcgtcgtg    1440
   acccatggcg atgcctgctt gccgaatatc atggtggaaa atggccgctt ttctggattc    1500
   atcgactgtg gccggctggg tgtggcggac cgctatcagg acatagcgtt ggctacccgt    1560
   gatattgctg aagagcttgg cggcgaatgg gctgaccgct tcctcgtgct ttacggtatc    1620
   gccgctcccg attcgcagcg catcgccttc tatcgccttc ttgacgagtt cttctga       1677
<210> 16
   <211> 583
   <212> DNA
   <213> Encephalomyocarditis virus
<400> 16
   ggcgcgcccc cctctccctc ccccccccct aacgttactg gccgaagccg cttggaataa 60
   ggccggtgtg cgtttgtcta tatgtgattt tccaccatat tgccgtcttt tggcaatgtg 120
   agggcccgga aacctggccc tgtcttcttg acgagcattc ctaggggtct ttcccctctc 180
   gccaaaggaa tgcaaggtct gttgaatgtc gtgaaggaag cagttcctct ggaagcttct 240
   tgaagacaaa caacgtctgt agcgaccctt tgcaggcagc ggaacccccc acctggcgac 300
   aggtgcctct gcggccaaaa gccacgtgta taagatacac ctgcaaaggc ggcacaaccc 360
   cagtgccacg ttgtgagttg gatagttgtg gaaagagtca aatggctctc ctcaagcgta 420
   ttcaacaagg ggctgaagga tgcccagaag gtaccccatt gtatgggatc tgatctgggg 480
   cctcggtgca catgctttac atgtgtttag tcgaggttaa aaaaacgtct aggccccccg 540
   aaccacgggg acgtggtttt cctttgaaaa acacgatgga tcc 583
<210> 17
   <211> 655
   <212> DNA
   <213> Enterovirus 71
<400> 17
   ggcgcgcccc cgaagtaact tagaagctgt aaatcaacga tcaatagcag gtgtggcaca 60
   ccagtcatac cttgatcaag cacttctgtt tccccggact gagtatcaat aggctgctcg 120
   cgcggctgaa ggagaaaacg ttcgttaccc gaccaactac ttcgagaagc ttagtaccac 180
   catgaacgag gcagggtgtt tcgctcagca caaccccagt gtagatcagg ctgatgagtc 240
   actgcaaccc ccatgggcga ccatggcagt ggctgcgttg gcggcctgcc catggagaaa 300
   tccatgggac gctctaattc tgacatggtg tgaagagcct attgagctag ctggtagtcc 360
   tccggcccct gaatgcggct aatcctaact gcggagcaca tgctcacaaa ccagtgggtg 420
   gtgtgtcgta acgggcaact ctgcagcgga accgactact ttgggtgtcc gtgtttcctt 480
   ttattcctat attggctgct tatggtgaca atcaaaaagt tgttaccata tagctattgg 540
   attggccatc cggtgtgcaa cagggcaatt gtttacctat ttattggttt tgtaccatta 600
   tcactgaagt ctgtgatcac tctcaaattc attttgaccc tcaacacaat caaac 655